(19) Europäisches Patentamt
European Patent Office
Office européen des brevets

(11) Veröffentlichungsnummer: **0 404 721 A2**

# (12) EUROPÄISCHE PATENTANMELDUNG

(21) Anmeldenummer: 90810427.6

(22) Anmeldetag: 12.06.90

(51) Int. Cl.[5]: C07D 405/04, C07D 409/04, A01N 43/36, //(C07D405/04, 307:00,207:00),(C07D409/04, 333:00,207:00)

(30) Priorität: 20.06.89 CH 2299/89

(43) Veröffentlichungstag der Anmeldung: 27.12.90 Patentblatt 90/52

(84) Benannte Vertragsstaaten: AT BE CH DE DK ES FR GB GR IT LI LU NL SE

(71) Anmelder: CIBA-GEIGY AG
Klybeckstrasse 141
CH-4002 Basel(CH)

(72) Erfinder: Huxley, Alica, Dr.
Margarethenstrasse 75
CH-4102 Binningen(CH)
Erfinder: Kunz, Walter, Dr.
Buchenstrasse 9
CH-4104 Oberwil(CH)
Erfinder: Ackermann, Peter, Dr.
Hangelimattweg 113
CH-4148 Pfeffingen(CH)
Erfinder: Sutter, Marius, Dr.
Klingental 5
CH-4058 Basel(CH)

## (54) Mikrobizide Mittel.

(57) Neue Benzothiophen- und Benzofuran-Derivate der allgemeinen Formel

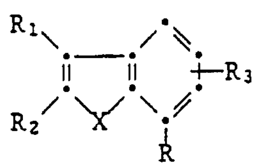

(I),

worin X Sauerstoff oder Schwefel, $R_1$ Wasserstoff, Halogen, $C_1$-$C_4$-Alkyl, $C_1$-$C_4$-Haloalkyl, $R_2$ Wasserstoff, Halogen, $C_1$-$C_4$-Alkyl, $C_1$-$C_4$-Haloalkyl, $R_3$ Wasserstoff, $C_1$-$C_4$-Alkyl, Halogen und R einen 4-Cyano-3-pyrrolrest bedeutet, dessen N-Atom einen der folgenden Substituenten trägt:

A: Wasserstoff oder CO-$R_4$, wobei $R_4$ unsubstituiertes $C_1$-$C_6$-Alkyl oder durch Halogen oder $C_1$-$C_3$-Alkoxy substituiertes $C_1$-$C_6$-Alkyl; $C_3$-$C_6$-Alkenyl, $C_3$-$C_6$-Alkinyl, unsubstituiertes $C_1$-$C_6$-Alkoxy oder durch Halogen oder $C_1$-$C_3$-Alkoxy substituiertes $C_1$-$C_6$-Alkoxy; $C_3$-$C_6$-Alkenyloxy, $C_3$-$C_6$-Cycloalkyl oder Tetrahydrofur-2-yl darstellt;

B: S-$R_5$, worin $R_5$ $C_1$-$C_3$-Haloalkyl bedeutet;

C: CH(Y)$R_6$, wobei $R_6$ für Wasserstoff oder $C_1$-$C_8$-Haloalkyl und Y für Hydroxy, Halogen oder OC(O)-$R_7$ stehen, worin $R_7$ $C_1$-$C_8$-Alkyl, $C_1$-$C_8$-Haloalkyl, $C_2$-$C_6$-Alkenyl, 2-Tetrahydrofuryl, 2-Tetrahydropyranyl oder $C_1$-$C_6$-Alkoxycarbonyl darstellen;

D: $CH_2$-Z, wobei Z für eine der Gruppen

EP 0 404 721 A2

a) 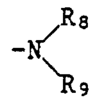 oder b) 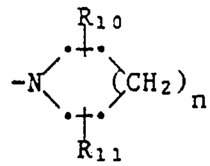 oder c) 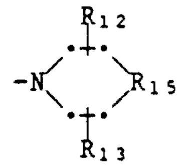

steht, worin $R_8$ und $R_9$ unabhängig voneinander Wasserstoff, unsubstituirtes oder durch Cyano, $C_1$-$C_6$-Alkoxycarbonyl substituiertes $C_1$-$C_6$-Alkyl; $C_3$-$C_6$-Alkenyl, $C_3$-$C_6$-Alkenyl, $C_3$-$C_7$-Cycloalkyl, unsubstituiertes oder durch Halogen, $C_1$-$C_6$-Alkyl, $C_1$-$C_6$-Haloalkyl und/oder $C_1$-$C_6$-Alkoxy substituiertes Phenyl bedeuten, mit der Massgabe, dass nur $R_8$ oder $R_9$ Wasserstoff sein kann; $R_{10}$ und $R_{11}$ unabhängig voneinander für Wasserstoff, $C_1$-$C_6$-Alkyl, $C_1$-$C_6$-Alkoxycarbonyl darstellen; $R_{12}$ und $R_{13}$ unabhängig voneinander für Wasserstoff, $C_1$-$C_6$-Alkyl oder $C_1$-$C_6$-Alkoxycarbonyl stehen; und $R_{15}$ Sauerstoff, Schwefel, $>C=O$ oder $>N-R_{14}$ bedeutet; worin $R_{14}$ Wasserstoff, $C_1$-$C_6$-Alkyl, Formyl; $C_1$-$C_6$-Akanoyl oder $C_1$-$C_6$-Alkoxycarbonyl darstellt; und n für eine der Zahlen 0 oder 1 steht.

Die neue Wirkstoffe dienen der Bekämpfung schädlicher Mikroorganismen, vor allem phytopathogener Pilze. Sie können zusammen mit geeigneten Formulierungshilfsstoffen als Mittel eingesetzt werden und eignen sich gleichfalls zur Verhütung des Befalls von Kulturpflanzen durch schädliche Mikroorganismen.

## Mikrobizide Mittel

Die vorliegende Erfindung betrifft neue substituierte Benzothiophen-und Benzofuran-Derivate, deren Herstellung, sowie mikrobizide Mittel, die als Wirkstoff mindestens eine dieser Verbindungen enthalten. Die Erfindung betrifft ferner die Herstellung der genannten Mittel und die Verwendung der neuen Wirkstoffe und Mittel zur Bekämpfung schädlicher Mikroorganismen, insbesondere pflanzenschädigender Pilze.

Die erfindungsgemässen Verbindungen haben die allgemeine Formel I

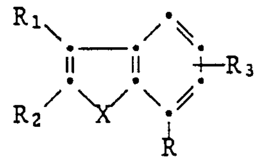 (I),

worin X Sauerstoff oder Schwefel, $R_1$ Wasserstoff, Halogen, $C_1$-$C_4$-Alkyl, $C_1$-$C_4$-Haloalkyl, $R_2$ Wasserstoff, Halogen, $C_1$-$C_4$-Alkyl, $C_1$-$C_4$-Haloalkyl, $R_3$ Wasserstoff, $C_1$-$C_4$-Alkyl, Halogen und R einen 4-Cyano-3-pyrrolrest bedeutet, dessen N-Atom einen der folgenden Substituenten trägt:

A: Wasserstoff oder CO-$R_4$, wobei $R_4$ unsubstituiertes $C_1$-$C_6$-Alkyl oder durch Halogen oder $C_1$-$C_3$-Alkoxy substituiertes $C_1$-$C_6$-Alkyl; $C_3$-$C_6$-Alkenyl, $C_3$-$C_6$-Alkinyl, unsubstituiertes $C_1$-$C_6$-Alkoxy oder durch Halogen oder $C_1$-$C_3$-Alkoxy substituiertes $C_1$-$C_6$-Alkoxy; $C_3$-$C_6$-Alkenyloxy, $C_3$-$C_6$-Cycloalkyl oder Tetrahydrofur-2-yl darstellt;

B: S-$R_5$, worin $R_5$ $C_1$-$C_3$-Haloalkyl bedeutet;

C: CH(Y)$R_6$, wobei $R_6$ für Wasserstoff oder $C_1$-$C_8$-Haloalkyl und Y für Hydroxy, Halogen oder OC(O)-$R_7$ stehen, worin $R_7$ $C_1$-$C_8$-Alkyl, $C_1$-$C_8$-Haloalkyl, $C_2$-$C_6$-Alkenyl, 2-Tetrahydrofuryl, 2-Tetrahydropyranyl oder $C_1$-$C_6$-Alkoxycarbonyl darstellen;

D: $CH_2$-Z, wobei Z für eine der Gruppen

a) 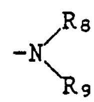 oder b) 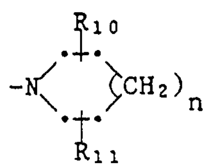 oder c) 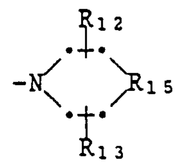

steht, worin $R_8$ und $R_9$ unabhängig voneinander Wasserstoff, unsubstituiertes oder durch Cyano, $C_1$-$C_6$-Alkoxycarbonyl substituiertes $C_1$-$C_6$-Alkyl; $C_3$-$C_6$-Alkenyl, $C_3$-$C_6$-Alkinyl, $C_3$-$C_7$-Cycloalkyl, unsubstituiertes oder durch Halogen, $C_1$-$C_6$-Alkyl, $C_1$-$C_6$-Haloalkyl und/oder $C_1$-$C_6$-Alkoxy substituiertes Phenyl bedeuten, mit der Massgabe, dass nur $R_8$ oder $R_9$ Wasserstoff sein kann; $R_{10}$ und $R_{11}$ unabhängig voneinander für Wasserstoff, $C_1$-$C_6$-Alkyl, $C_1$-$C_6$-Alkoxycarbonyl darstellen; $R_{12}$ und $R_{13}$ unabhängig voneinander für Wasserstoff, $C_1$-$C_6$-Alkyl oder $C_1$-$C_6$-Alkoxycarbonyl stehen; und $R_{15}$ Sauerstoff, Schwefel, $>C=O$ oder $>N-R_{14}$ bedeutet, worin $R_{14}$ Wasserstoff, $C_1$-$C_6$-Alkyl, Formyl, $C_1$-$C_6$-Alkanoyl oder $C_1$-$C_6$-Alkoxycarbonyl darstellt; und n für eine der Zahlen 0 oder 1 steht.

Unter dem Begriff Alkyl selbst oder als Bestandteil eines anderen Substituenten sind je nach der Zahl der angegebenen Kohlenstoffatome beispielsweise folgende Gruppen zu verstehen: Methyl, Ethyl, Propyl, Butyl, Pentyl, Hexyl usw., sowie ihre Isomeren, wie z.B. Isopropyl, Isobutyl, tert.-Butyl, Isopentyl usw.. Haloalkyl steht für einen einfach bis perhalogenierten Alkylsubstituenten, wie z.B. $CH_2Cl$, $CHCl_2$, $CCl_3$, $CH_2Br$, $CHBr_2$, $CBr_3$, $CH_2F$, $CHF_2$, $CF_3$, $CCl_2F$, $CCl_2$-$CHCl_2$, $CH_2CH_2F$, $CJ_3$ usw. Unter Halogen soll hier und im folgenden Fluor, Chlor, Brom oder Jod, vorzugsweise Fluor, Chlor oder Brom, verstanden werden. $C_3$-$C_6$-Alkenyl steht für einen ungesättigten, aliphatischen Rest mit einer oder mit mehreren Doppelbindungen, so z.B. für Propenyl-(1), Allyl, Butenyl-(1), Butenyl-(2), Butenyl-(3), $CH_3CH=CHCH=CH$- usw. Unter Alkinyl sind ungesättigte, aliphatische Reste und maximal 6 Kohlenstoffatomen zu verstehen, z.B. Propargyl, Butinyl-(2), Butinyl-(3) usw.

Die Verbindungen der Formel I sind unter Normalbedingungen stabile Oele, Harze oder überwiegend kristalline Feststoffe, die sich durch äusserst wertvolle mikrobizide Eigenschaften auszeichnen. Sie lassen sich beispielsweise auf dem Agrarsektor oder verwandten Gebieten präventiv und kurativ zur Bekämpfung pflanzenschädigender Mikroorganismen einsetzen. Die erfindungsgemässen Wirkstoffe der Formel I zeichnen sich in weiten Anwendungskonzentrationen durch eine hohe fungizide Aktivität und problemlose Anwendung aus.

Von den Verbindungen der Formel I werden aufgrund ihrer mikrobiziden Wirkung jene bevorzugt, bei welchen der Pyrrolrest am N-Atom einen der folgenden Reste trägt:

Wasserstoff oder CO-$R_4$, wobei $R_4$ unsubstituiertes $C_1$-$C_6$-Alkyl oder durch Halogen oder $C_1$-$C_3$-Alkoxy substituiertes $C_1$-$C_6$-Alkyl; $C_3$-$C_6$-Alkenyl, $C_3$-$C_6$-Alkinyl, unsubstituiertes $C_1$-$C_6$-Alkoxy oder durch Halogen oder $C_1$-$C_3$-Alkoxy substituiertes $C_1$-$C_6$-Alkoxy; $C_3$-$C_6$-Alkenyloxy, $C_3$-$C_6$-Cycloalkyl oder Tetrahydrofur-2-yl darstellt.

Von dieser Gruppe sind insbesondere jene bevorzugt, bei welchen das N-Atom des Pyrrols einen der folgenden Resten trägt:

Wasserstoff oder $COR_4$, wobei $R_4$ unsubstituiertes $C_1$-$C_4$-Alkyl oder durch Chlor, Brom oder $C_1$-$C_3$-Alkoxy substituiertes $C_1$-$C_4$-Alkyl; $C_3$-$C_4$-Alkenyl, $C_3$-$C_4$-Alkinyl, unsubstituiertes $C_1$-$C_4$-Alkoxy, oder durch Chlor, Brom oder $C_1$-$C_3$-Alkoxy substituiertes $C_1$-$C_4$-Alkoxy; $C_3$-$C_4$-Alkenyloxy, $C_3$-$C_6$-Cycloalkyl oder Tetrahydrofur-2-yl, darstellt.

Von den zuvor genannten Wirksubstanzen sind jene hervorzuheben, bei welchen der Pyrrolrest am N-Atom ein Wasserstoffatom trägt, und von der letztgenannten Gruppe insbesondere jene, bei welchen $R_1$ Wasserstoff, Halogen, $C_1$-$C_2$-Alkyl oder $C_1$-$C_2$-Haloalkyl, $R_2$ Wasserstoff oder Halogen und $R_3$ Wasserstoff bedeuten.

Von diesen Verbindungen sind als Mikrobizide jene besonders aktiv, bei welchen $R_1$ Wasserstoff oder Halogen und $R_2$ Wasserstoff bedeuten.

Als Einzelverbindungen dieser Gruppe sind das 3-(Brombenzothiophen-7-yl)-4-cyanopyrrol und das 3-(Benzothiophen-7-yl)-4-cyanopyrrol wegen ihrer Aktivität zu unterstreichen.

Die Verbindungen der Formel I werden erfindungsgemäss hergestellt:

a) in alkalischem Medium durch cyclisierende Michael-Addition einer Cyanoacrylsäure, eines Cyanoacrylsäureesters oder eines Cyanoacrylsäureamids der Formel IIa an p-Toluolsulfonylmethylisocyanid unter Abspaltung von p-Toluolsulfinsäure bzw. ihres Salzes in einem organischen Lösungsmittel:

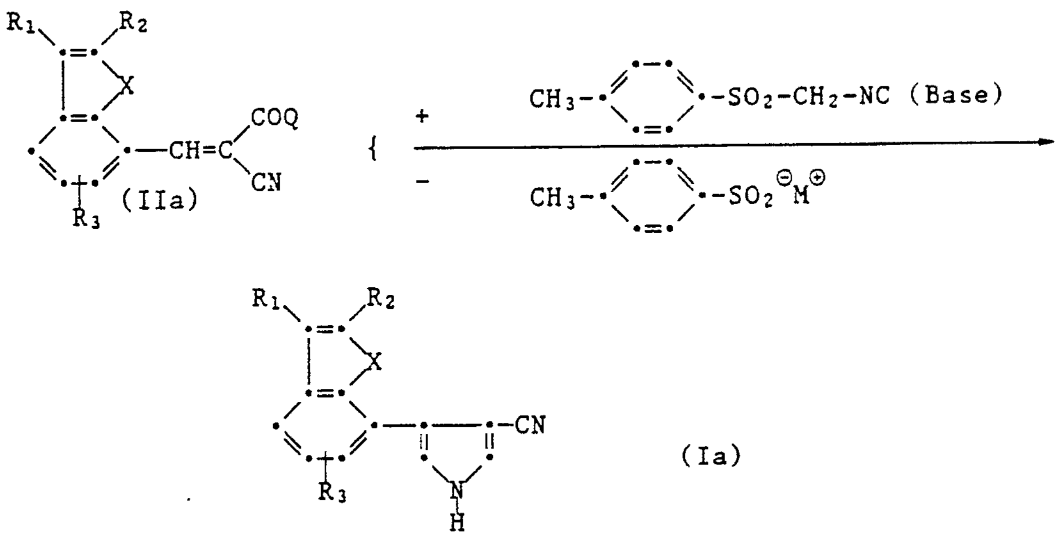

(IIa)
(Ia)

wobei $M^{\oplus}$ ein Alkali- oder Erdalkaliion und Q $OR_{16}$ oder $NH_2$ bedeuten und $R_{16}$ für Wasserstoff oder $C_1$-$C_4$-Alkyl steht,

b) durch anschliessende Acylierung der Verbindung Ia mit Verbindungen der Formel III in Gegenwart eines säurebindenden Mittels und gegebenenfalls eines Katalysators in einem organischen Lösungsmittel:

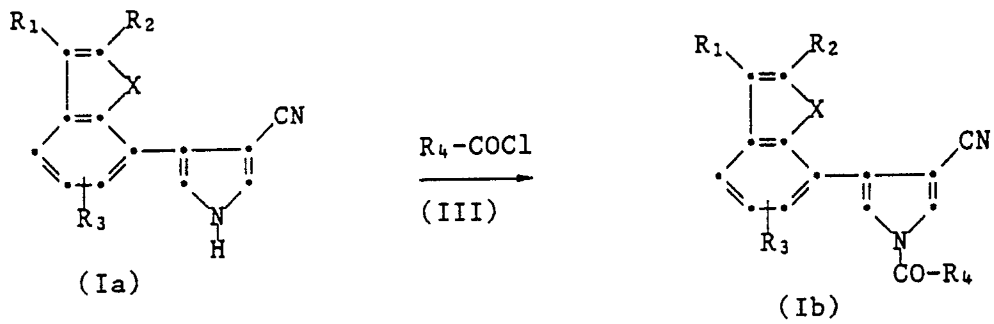


(Ia) (Ib)

wobei $R^4$ die eingangs unter Formel I angegebenen Bedeutungen hat,

oder

c) durch Sulfenylierung der Verbindung der Formel Ia mit einem reaktionsfähigen Säurederivat einer Sulfensäure der Formel IV

$R_5$S-OH (IV)

am Pyrrol-Stickstoffatom in Gegenwart eines säurebindenden Mittels gegebenenfalls in einem organischen Lösungsmittel, wobei $R_5$ die eingangs unter Formel I angegebenen Bedeutungen hat,

oder

d) durch Umsetzung der Verbindung der Formel Ia mit einem Aldehyd der Formel V

$R_6$-CHO (V)

zu einem Hydroxyderivat der Formel Ic

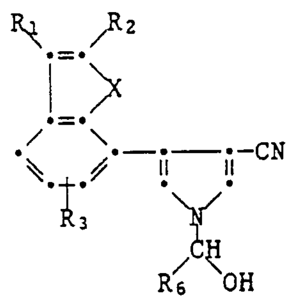

(Ic)

und letzteres weiter durch Austausch der OH-Gruppe gegen einen andern Rest Y in eines der übrigen Produkte der Formel I überführt, wobei dieser Austausch dadurch erfolgt, dass man eine Verbindung der Formel Ic entweder mit einer Säure der Formel VI

$R_7$-COOH (VI)

oder vorzugsweise mit einem ihrer reaktionsfähigen Säurederivate, insbesondere Säurehalogenide, wie z.B. Säurechlorid oder Säurebromid oder mit ihrem Säurehanhydrid in ein Acyloxy-Produkt der Formel Id

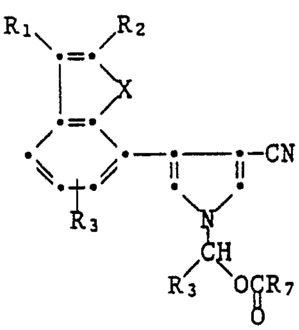

(Id)

überführt, oder dadurch, dass man die OH-Gruppe in einer Verbindung der Formel Ic zuerst auf übliche Weise gegen ein Halogen, vorzugsweise Chlor oder Brom, austauscht und die Verbindung der Formel Ie

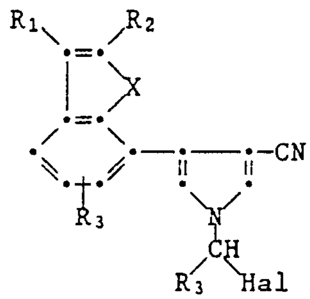

(Ie)

erhält und dann weiter das halogenierte Produkt durch Reaktion mit einem Salz der Formel VII

$R_7$-COO$^{\oplus}$ M$'^{\oplus}$ (VII)

in eine Verbindung der Formel Id überführt, wobei die Substituenten in den Formeln Ic, Id, Ie, V, VI und VII die unter Formel I angegebene Bedeutung haben, sowie Hal für Halogen und M$'^{\oplus}$ für ein Metallkation, vorzugsweise für ein Erdalkali- oder insbesondere Alkalimetallkation, wie z.B. Ca$^{\oplus\oplus}$, Mg$^{\oplus}$, Na$^{\oplus}$ oder K$^{\oplus}$, stehen, oder

e) durch Umsetzung der Verbindung der Formel Ia mit einer Verbindung der Formel VIII

H-Z (VIII)

in welcher Z die eingangs unter Formel I angegebene Bedeutungen hat, entweder in einem protischen Lösungsmittel bei Temperaturen von 0° bis 120° C, vorzugsweise bei 20° C bis Rückflusstemperatur, in Gegenwart eines basischen oder sauren Reaktionskatalysators mit Formaldehyd; oder in einem aprotischen Lösungsmittel oder in Gegenwart eines basischen Reaktionskatalysators bei Temperaturen von 0° bis 120° C, vorzugsweise 20° bis 80° C, mit 1,3,5-Trioxan oder Paraformaldehyd.

In der Stufe a) der geschilderten Reaktionsfolge können erfindungsgemäss die Cyanoacrylsäure oder ihre Ester der Formel IIa durch ein Acrylnitril der Formel IIb ersetzt werden:

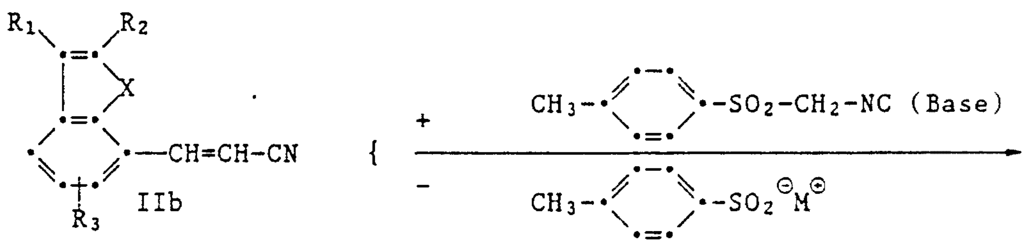


Ia

Reaktionsschritt (a):

Die p-Tolylsulfonyl-Gruppe steht hier stellvertretend für eine ganze Reihe von Gruppen, die in der Lage sind, die Methylengruppe im Methylisocyanid-Rest für eine Reaktion im Sinne einer Michael-Addition zu aktivieren. Weitere bevorzugte Beispiele für derartig aktivierende Gruppen sind Benzolsulfonyl-, p-Chlorbenzolsulfonyl, Niederalkylsulfonyl wie Mesyl.

Die Cycloaddition wird in Gegenwart einer geeigneten nukleophilen Base durchgeführt. Als Basen eignen sich Alkalimetallhydride wie Natriumhydrid oder Alkali- bzw. Erdalkalicarbonate, wie $Na_2CO_3$, $K_2CO_3$ oder Alkalialkoholate, wie $(CH_3)_3CO^{\ominus}K^{\oplus}$, $NaOCH_3$ und andere. Die Base wird vorteilhafterweise in mindestens äquimolarer Menge, bezogen auf die Ausgangssstoffe, eingesetzt.

Für die Cycloaddition ist es zweckmässig, die Reaktion in einem reaktionsinerten Lösungsmittel durchzuführen. Dafür kommen beispielsweise folgende, bevorzugt wasserfreie Lösungsmittel in Frage: Aromatische und aliphatische Kohlennwasserstoffe wie Benzol, Toluol, Xylole, Petrolether, Ligroin, Cyclohexan; Ether und etherartige Verbindungen wie Dialkylether (Diethylether, Diisopropylether, t-Butylmethylether usw.), Dimethoxymethan, Dioxan, Tetrahydrofuran, Anisol; Alkohole, wie Methanol oder Ethanol; Sulfone wie Dimethylsulfoxid; Dimethylformamid und Gemische solcher Lösungsmittel untereinander.

Die Cycloaddition wird im allgemeinen bei Temperaturen von -30° bis +120° C, bevorzugt bei -30° bis +50° C bzw. am Siedepunkt des Lösungsmittels oder Lösungsmittelgemisches, durchgeführt.

Bei der Wahl geeigneter Basen lässt sich die Cycloaddition vorteilhafterweise auch in wässrigem Medium ausführen. Als Basen eignen sich in diesen Fällen wasserlösliche anorganische und organische

Basen, insbesondere Alkalihydroxide wie LiOH, NaOH oder KOH und Ammoniumbasen, z.B. Tetraalkylammoniumhydroxide wie $(CH_3)_4NOH$. Die Base wird in mindestens äquimolarer Menge, bezogen auf die Ausgangsstoffe, eingesetzt. Bei der Verwendung wässriger Basen führt man die Reaktion vorteilhafterweise in einem heterogenen Zweiphasensystem durch.

Für die organische, mit Wasser nicht mischbare Phase kommen z.B. folgende Lösungsmittel in Frage: Aliphatische und aromatische Kohlenwasserstoffe, wie Pentan, Hexan, Cyclohexan, Petrolether, Ligroin, Benzol, Toluol, Xylole usw., halogenierte Kohlenwasserstoffe, wie Dichlormethan, Chloroform, Tetrachlorkohlenstoff, Ethylendichlorid, 1,2-Dichlorethan, Tetrachlorethylen usw., oder aliphatische Ether, wie Diethylether, Diisopropylether, t-Butylmethylether usw.

Zur Beschleunigung der Reaktionsgeschwindigkeit kann bei dieser Durchführungsart die Gegenwart eines Phasentransfer-Katalysators von Vorteil sein. Beispiele derartiger Katalysatoren sind: Tetraalkylammoniumhalogenide, -hydrogensulfate oder -hydroxide wie Tetrabutylammoniumchlorid, -bromid, -jodid; Triethylbenzylammoniumchlorid, -bromid; Tetrapropylammoniumchlorid, -bromid-, -jodid usw. Als Phasentransfer-Katalysatoren kommen auch Phosphonium-Salze in Betracht.

Die Phasentransfer-katalysierte Cycloaddition kann bei Temperaturen von 0° bis 80° C, vorzugsweise 10° bis 50° C bzw. beim Siedepunkt des Lösungsmittelgemisches, durchgeführt werden.

Die Cycloaddition kann in den beschriebenen Ausführungsarten bei Normaldruck erfolgen; die Reaktionsdauer beträgt im allgemeinen 1 bis 16 Stunden, bei Phasentransferkatalyse 0,5 bis 10 Stunden.

Reaktionsschritt b)

Die Acylierung der Verbindung Ia wird unter üblichen, dem Fachmann bekannten Bedingungen durchgeführt.

Geeignete Lösungs- oder Verdünnungsmittel sind beispielsweise aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Dioxan, Tetrahydrofuran; Nitrile wie Acetonitril, Propionitril; Ketone wie Aceton Diethylketon, Methylethylketon; und Gemische solcher Lösungsmittel untereinander. Bevorzugt sind Tetrahydrofuran oder Dioxan.

Als säurebindende Mittel eignen sich anorganische Basen, z.B. Oxide, Hydroxide, Carbonate oder Hydrogencarbonate von Alkali- oder Erdalkalimetallen sowie Alkalihydride oder Alkyliacetate, ferner organische Basen, z.B. tertiäre Amine wie Trialkylamine (Trimethylamin, Triethylamin usw.), Pyridin oder Pyridinbasen (4-Dimethylaminopyridin, 4-Pyrrolidylaminopyridin). Bevorzugt sind Trialkylamine wie Trimethylamin oder Triethylamin.

Die Reaktionstemperatur ist je nach den Reaktionsbedingungen variabel. Sie liegt im allgemeinen zwischen -25° C und 100° C, vorzugsweise zwischen -10° und 75° C.

Reaktionsschritt c)

Für die Sulfenylierungsreaktion eignen sich als reaktionsfähige Sulfensäurederivate z.B. die Niederalkylester und bevorzugt die Sulfonsäurehalogenide, insbesondere die -chloride und -bromide und von diesen wiederum besonders die -chloride. Niederalkyl steht hierbei für $C_1$-$C_6$-Alkyl.

Als Basen können sowohl organische als auch anorganische Basen mit Erfolg eingesetzt werden. Geeignete anorganische Basen sind z.B. Alkali- und Erdalkalicarbonate, wie Natriumcarbonat, Kaliumcarbonat, Calciumcarbonat usw. Geeignete organische Basen sind z.B. tertiäre Amine wie Trialkylamine (Triethylamin, Methyldiethylamin), N,N-Dimethoxycyclohexylamin, N-Methylpiperidin, N,N-Dimethylanilin oder Pyridine. Bevorzugt werden die Trialkylamine. Vorteilhafterweise wird die Base in stöchiometrischer Menge oder im Ueberschuss, z.B. bis zu 100 % Ueberschuss, in Bezug auf das Pyrrol Ia eingesetzt. Auch das reaktionsfähige Derivat der Sulfesäure IV wird entweder in stöchiometrischer Menge oder im Ueberschuss zugesetzt.

Die Sulfenylierungs-Reaktion kann in An- oder Abwesenheit, vorzugsweise in Anwesenheit eines reaktionsinerten Lösungsmittels oder Lösungsmittelgemisches, durchgeführt werden. Prinzipiell eignen sich für diese Umsetzung die üblichen organischen Lösungsmittel, sofern sie keine reaktiven Wasserstoffatome enthalten. Geeignet sind beispielsweise aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Ethylenglykol- und Diethylenglykoldiether und -monoether mit je 1 - 4 C-Atomen in den Alkylteilen, wie Ethylenglykoldimethyl-, -diethyl- und -di-n-butylether, Diethylenglykoldiethyl- und -di-n-butylether, Ethylenglykolmonomethylether und Diethylenglykolmonomethylether; Furan, Dimethoxyethan, Dioxan. Tetrahydrofuran, Anisol; Sulfone wie Dimethylsulfoxid; Ketone wie Aceton, Methylethylketon; Ester wie Ethylacetat, Propylacetat; Butylacetat und Gemische solcher Lösungsmittel untereinander. In einigen Fällen kann auch das Sulfenylierungsreagenz der Formel IV selbst als Lösungsmittel fungieren.

Zur Beschleunigung der Reaktionsgeschwindigkeit kann unter Umständen ein Reaktionskatalysator wie 4-Dimethylaminopyridin zugesetzt werden.

Zur Sulfenylierungsreaktion wird im allgemeinen bei Temperaturen zwischen -30° bis +100° C, vorzugsweise -10° bis +20° C, durchgeführt. Die Reaktionszeiten liegen dabei erfahrungsgemäss etwa zwischen 0,5 h bis 20 h. Durch Zugabe eines Reaktionskatalysators kann eine Verkürzung der Reaktionsdauer auf weniger als 0,5 h erreicht werden.

Reaktionsschritt d)

Die Reaktion der Verbindung der Formel Ia mit Aldehyden der Formel V kann in An- oder Abwesenheit reaktionsinerter Lösungsmittel oder Lösungsmittelgemische durchgeführt werden. Dazu eignen sich beispielsweise aromatische Kohlenwasserstoffe, wie Benzol, Toluol oder Xylole; halogenierte Kohlenwasserstoffe, wie Chlorbenzol; aliphatische Kohlenwasserstoffe wie Petrolether; Ether und etherartige Verbindungen, wie Dialkylether (Diethylether, Diisopropylether, tert.-Butylmethylether usw.), Furan, Dimethoxyethan, Dioxan, Tetrahydrofuran; Dimethylformamid usw..

Die Umsetzung von Verbindungen der Formel Ia mit Verbindungen der Formel V wird vorteilhafterweise ohne Lösungsmittel, jedoch mit einer überschüssigen Menge des Aldehyds der Formel V durchgeführt. Je nach Art des Aldehyds arbeitet man in Lösung oder in der Schmelze. Hierbei wirkt sich die Zugabe saurer oder basischer Katalysatoren günstig auf die Reaktionsgeschwindigkeit aus. Als saure Katalysatoren kommen z.B. wasserfreie Halogenwasserstoffe und Mineralsäuren, wie HCl, HBr oder $H_2SO_4$ aber auch konzentrierte Salzsäure in Frage. Als basische Katalysatoren können z.B. Trialkylamine (Trimethylamin, Triethylamin, Dimethylethylamin, usw.), Alkali- und Erdalkalicarbonate (wie $Na_2CO_3$, $BaCO_3$, $MgCO_3$, $K_2CO_3$, usw.) oder Alkalialkoholate (wie $NaOCH_3$, $NaOC_2H_5$, KO(iso-$C_3H_7$), KO(t.-Butyl) eingesetzt werden. Die Temperaturen liegen bei dieser Umsetzung im allgemeinen zwischen 0° und 200° C, vorzugsweise 0° und 160° C, und die Reaktionsdauer beträgt 1 bis 24 Stunden, insbesondere 1 bis 4 Stunden.

Der Austausch der freien Hydroxylgruppe in den Verbindungen der Formel Ic gegen eine Gruppe Y wird bevorzugt in einem reaktionsinerten Lösungsmittel durchgeführt. Beispiele solcher Lösungsmittel sind: Aromatische und aliphatische Kohlenwasserstoffe wie Benzol, Toluol, Xylole, Petrolether, Ligroin oder Cyclohexan; halogenierte Kohlenwasserstoffe wie Chlorbenzol, Methylenchlorid, Ethylenchlorid, Chloroform, Tetrachlorkohlenstoff oder Tetrachlorethylen; Ether und etherartige Verbindungen wie Diethylether, Diisopropylether, t.-Butylmethylether, Dimethoxyethan, Dioxan, Tetrahydrofuran oder Anisol; Ester wie Ethylacetat, Propylacetat oder Butylacetat; Nitrile wie Acetonitril; oder Verbindungen wie Dimethylsulfoxid, Dimethylformamid und Gemische solcher Lösungsmittel untereinander.

Die Einführung der Gruppe Y erfolgt nach üblichen Methoden. Bedeutet Y Chlor, so wird als Reagenz z.B. Phosphoroxychlorid, Phosphortrichlorid, Phosphorpentachlorid oder vorzugsweise Thionylchlorid eingesetzt. Man arbeitet im allgemeinen bei Temperaturen von 0° bis 120° C. Im Falle von Y = Brom verwendet man bevorzugt Phosphortribromid oder Phosphorpentabromid und führt die Reaktion bei 0° bis 50° C durch. Steht Y für die Gruppe -O-C(O)-$R_4$ so wird als Reagenz üblicherweise das entsprechende Säurehalogenid, insbesondere Säurechlorid, eingesetzt. Hierbei ist es zweckmässig, wenn die Reaktion bei Temperaturen von -20° bis +50° C, vorzugsweise -10° bis +30° C und in Gegenwart einer schwachen Base wie Pyridin oder Triethylamin durchgeführt wird. Ferner können als Katalysatoren 4-Dialkylaminopyridine wie 4-Dimethyl- oder 4-Diethylaminopyridin zur Beschleunigung der Reaktion zugesetzt werden.

Die Umsetzung von Verbindungen der Formel Ie mit Salzen der Formel VII erfolgt üblicherweise in Gegenwart eines üblichen inerten Lösungsmittels oder Lösungsmittelgemisches. Beispiele solcher Lösungsmittel sind: Aromatische und aliphatische Kohlenwasserstoffe wie Benzol, Toluol, Xylole, Petrolether, Ligroin oder Cyclohexan; Ether und etherartige Verbindungen wie Dialkylether z.B. Diethylether, Diisopropylether, T-Butylmethylether, Dimethoxyethan, Dioxan, Tetrahydrofuran oder Anisol; Ester wie Ethylacetat, Propylacetat oder Butylacetat; Nitrile wie Acetonitril; oder Verbindungen wie Dimethylsulfoxid, Dimethylformamid und Gemische solcher Lösungsmittel untereinander.

Die Zugabe katalytischer Mengen eines Kronenethers, wie z.B. 18-Krone-6 oder oder 15-Krone-5 wirken

sich bei dieser Umsetzung günstig auf den Reaktionsverlauf aus. Die Reaktionstemperaturen liegen im allgemeinen zwischen 0° und 150°C, vorzugsweise 20° bis 80°C. Die Reaktionsdauer beträgt 1 bis 24 Stunden.

In einer bevorzugten Ausführungsform wird die Herstellung von Verbindungen der Formel Id, insbesondere jener mit $R_3$ = $CCl_3$ oder $R_3$ = H, ausgehend von Verbindungen der Formel Ia, im sogenannten Eintopfverfahren durchgeführt. Hierbei arbeitet man vorteilhafterweise in einem der oben genannten inerten Lösungs- oder Verdünnungsmittel, besonders geeignet sind z.B. etherartige Verbindungen, wie Tetrahydrofuran, und in Gegenwart einer schwachen Base, wie Trialkylamin (Triethylamin) oder Pyridin. Als Reagenzien werden Chloral oder Paraformaldehyd eingesetzt. Die Reaktion kann durch Zugabe eines Katalysators wie z.B. 1,8-Diazabicyclo[5.4.0]-undec-7en [DBU] beschleunigt werden. Die Temperaturen liegen bei dieser ersten Reaktionsstufe bei -20° bis +100°C, bevorzugt 0° bis 50°C, und die Reaktionszeit bei 0,5 bis 2 Stunden. Es entsteht intermediär ein Hydroxyderivat der Formel Ic, das nicht isoliert wird, sondern in derselben Reaktionslösung bei -30° bis +30°C, vorzugsweise -10° bis 0°C und in Gegenwart katalytischer Mengen eines 4-Dialkylaminopyridins, vorzugsweise 4-Dimethylaminopyridin, mit einer Verbindung der Formel VI umgesetzt wird. Die Reaktionsdauer für diese 2. Stufe liegt bei 0,5 bis 16 Stunden.

Die Ausgangssubstanzen der Formeln V, VI und VII sind allgemein bekannt oder werden nach an sich bekannten Methoden hergestellt.

Reaktionsschritt e)

Die Umsetzung der Verbindung der Formel Ia mit einer Verbindung der Formel VIII wird vorzugsweise in einem geeigneten reaktionsinerten Lösungsmittel durchgeführt. Geeignete protische Lösungsmittel sind z.B.: Wasser, Alkohole (insbesondere Alkanole, wie Methanol, Ethanol, Isopropanol, n-Propanol usw.) oder Carbonsäuren (insbesondere Alkancarbonsäuren, wie Ameisensäure, Essigsäure, Propionsäure usw.). Arbeitet man in einem protischen Lösungsmittel, so können als Reaktionskatalysatoren z.B. folgende Substanzen eingesetzt werden: Organische Basen wie z.B. 1,8-Diazabicyclo[5.4.0]-undec-7-en, tertiäre Amine wie Trialkylamine, (Trimethylamin, Triethylamin, Dimethylethylamin, usw.), Triethylendiamin, Piperidin, Pyridin, 4-Dimethylaminopyridin, 4-Pyrrolidylpyridin usw. oder anorganische Basen wie die Oxide, Hydroxide, Hydride, Carbonate, Hydrogencarbonate und Alkoholate von Alkali- oder Erdalkalimetallen (z.B. $Na_2CO_3$, $BaCO_3$, $MgCO_3$,$K_2CO_3$, $CaHCO_3$, $NaOCH_3$, $NaOC_2H_5$, KO(iso-$C_3H_7$), KO(t.-Butyl), NaH, CaO usw.). Organische Säuren wie z.B. Carbonsäuren (Essigsäure, Ameisensäure, Propionsäure, usw.) aliphatische und aromatische Sulfonsäuren wie Methansulfonsäure, Ethansulfonsäure, Toluolsulfonsäure, Benzolsulfonsäure, usw. Anorganische Säuren wie Mineralsäuren, z.B. Phosphorsäure, Schwefelsäure, Salpetersäure oder Halogenwasserstoffsäuren (Chlorwasserstoffsäure, Bromwasserstoffsäure, Jodwasserstoffsäure oder Fluorwasserstoffsäure). Säuren oder Basen werden bei dieser Reaktionsvariante vorteilhafterweise in katalytischen Mengen zugesetzt. Im allgemeinen genügt bereits ein Ueberschuss des Amins der Formel VIII. Der Formaldehyd wird bei dieser Variante vorzugsweise in Form seiner wässrigen Lösung (Formalin) oder als Trimeres (1,3,5-Trioxan) oder als Polymeres (Paraformaldehyd) eingesetzt.

Als aprotische Lösungsmittel eignen sich z.B. aliphatische oder aromatische Kohlenwasserstoffe wie Benzol, Toluol, Xylole, Petrolether, Ligroin, Benzine oder Cyclohexan, Ether und etherartige Verbindungen wie Diethylether, Diisopropylether, Tert.-Butylmethylether, Dimethoxyethan, Tetrahydrofuran, Dioxan oder Anisol, Ester wie Ethylacetat, Propylacetat oder Butylacetat, oder Verbindungen wie Dimethylformamid, Dimethylsulfoxid und Gemische solcher Lösungsmittel untereinander. Als Katalysa toren werden z.B. die oben aufgeführten Basen eingesetzt. In diesem Reaktionsschritt wird der Formaldehyd vorzugsweise als 1,3,5-Trioxan oder Paraformaldehyd eingesetzt.

Die Sulfensäuren der Formel IV und die Amine der Formel VIII sind bekannt oder sind nach an sich bekannten Methoden herstellbar.

Bei der Herstellung der Cyanoacrylsäure ihrer Ester oder der Amide der Formel IIa geht man von dem Aldehyd der Formel IX aus, das in der üblichen, dem Fachmann bekannten Weise mit Cyanessigsäure, Cyanessigsäureester oder Cyanessigsäureamid kondensiert wird [Org. React. 15, 204-599 (1967)].

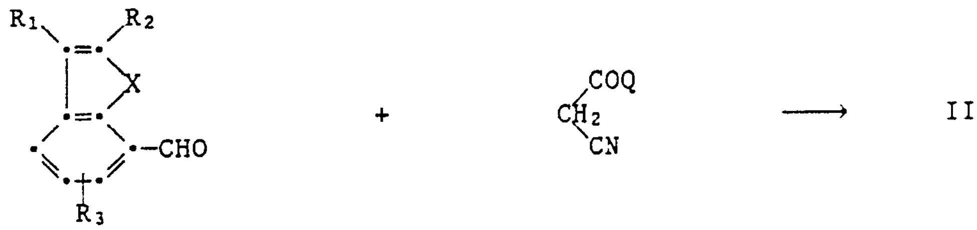


IX

Die 7-Benzofuran-carbaldehyde der Formel IXa

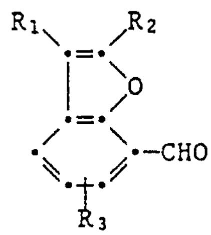

IXa

sind zum Teil bekannt [J. Chem. Perk. Transl. 2(a) 1479-1485, Tetrahedron Letters (24), 45, 5023-5024 (1983)] und können allgemein in Analogie zu den hierin publizierten Methoden hergestellt werden.

Die neuen 7-Benzothiophen-carbaldehyde der Formel IXb

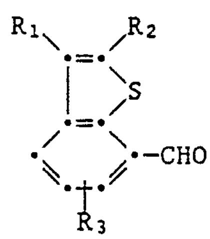

IXb

können in Analogie zu den zuvor erwähnten Methoden hergestellt werden, welche zu den 7-Benzofurancarbaldehyden führen. Dabei werden die durch Halogenierung der 7-Methylbenzothiophene der Formel X erhältlichen 7-Halomethylbenzothiophene der Formel XV entweder mit Hexamethylentetramin (Sommelet-Reaktion) oder mit einem Nitroalkan und mit einem Alkalialkoholat umgesetzt:

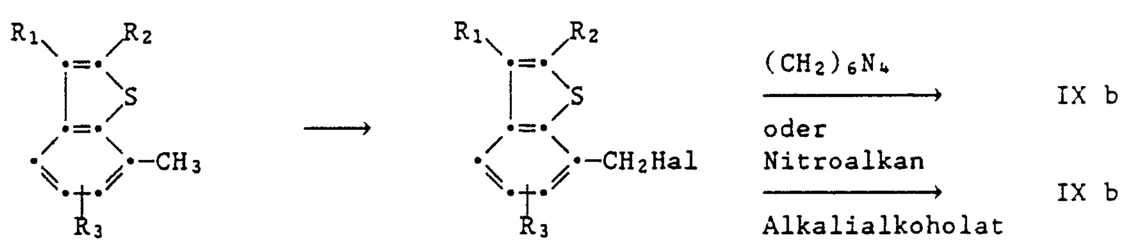


X XV.

Bei der Bromierung von X mit N-Bromsuccinimid in grossem Ueberschuss ist das 3-Brom-7-brommethylbenzothiophen direkt erhältlich, das seinerzeit übrigens durch Umsetzung mit 1 Mol Wasserstoff in Gegenwart von Pd-Katalysator in das 7-Brommethylbenzothiophen überführbar ist.

7-Methyl-benzofurane und 7-Methylbenzothiophene sind aus der DE-OS 3,302814, aus dem Bull. Soc. Chim. Fr. 1962; 30-34 und aus dem Bull. Soc. Chim. Fr. 1961; 2410-2417; bekannt.

Die Herstellung der Verbindungen der Formel X kann allgemein nach den in diesen Referenzen beschriebenen Methoden erfolgen.

Die neuen Cyano-acrylsäurederivate der Formel IIa sind wertvolle Zwischenprodukte für die Herstellung der mikrobiziden Verbindungen der Formel I und sind ebenfalls Gegenstand der vorliegenden Anmeldung.

Bei der Herstellung der Acrylnitrile der Formel IIb geht man von den, durch Reduktion der entsprechenden Nitroverbindungen erhältlichen 7-Aminobenzofuranen der Formel XIa oder 7-Aminobenzothiophenen der Formel XIb aus, welche in der üblichen, dem Fachmann bekannten Weise zum Diazoniumsalz der Formel XII umgesetzt werden:

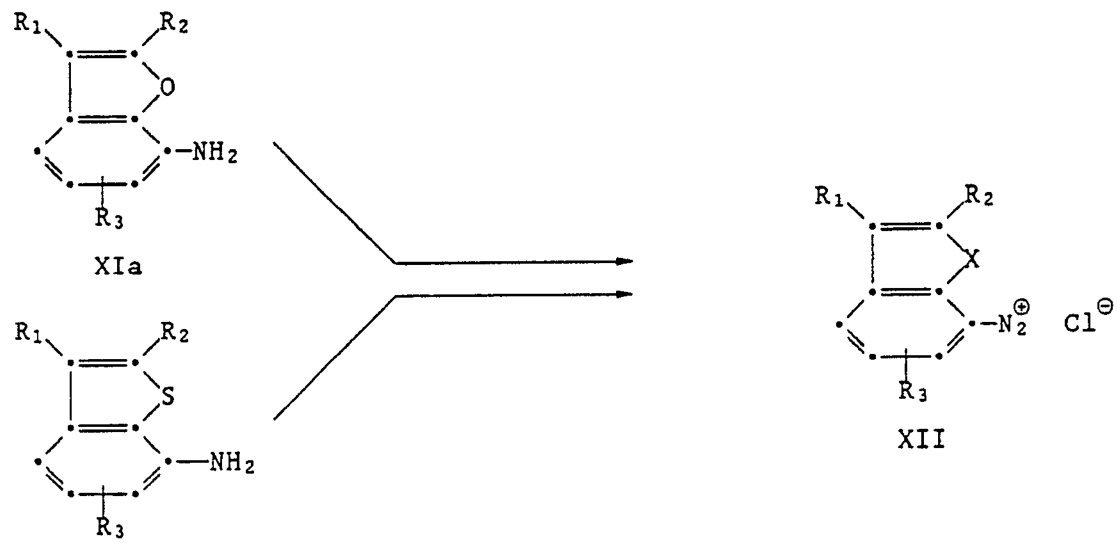

XIa

XIb

XII

Dann lässt man das Diazoniumsalz der Formel XII mit dem Acrylnitril der Formel XIII in Gegenwart von Cu(I)-Chlorid in einem wässrigen Reaktionsmedium mit einem Dialkylketon als Lösungsvermittler zu dem Anlagerungsprodukt der Formel XIV reagieren:

XII + $CH_2=CH-CN$ (XIII)

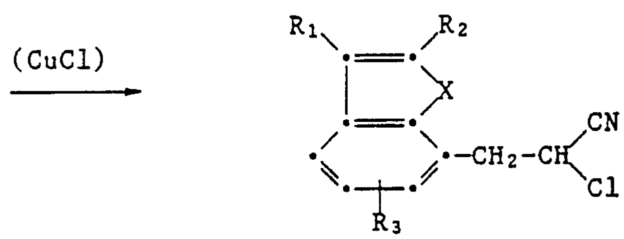


XIV

Anschliessend wird durch Umsetzung der Verbindung der Formel XIV mit einem säurebindenden Mittel in einem inerten organischen Lösungsmittel HCl abgespalten und dabei ein Acrylsäurenitril (Formel IIb) erhalten, wobei das Produkt ein cis/trans-Isomerengemisch darstellt, das chromatographisch in üblicher Weise getrennt werden kann:

$$XIV \xrightarrow{\text{Base}} IIb$$

Die durchgeführte Umsetzung des Diazoniumsalzes (Formel XII) mit dem Acrylnitril (Formel XIII) stellt eine Abwandlung der normalen "Sandmeyer"-Methode durch Anwendung der Bedingungen der "Meerwein"-Reaktion von aromatischen Diazonium-Verbindungen auf α-,β-ungesättigte Carbonyl-Verbindungen dar, wodurch der Ersatz der Diazoniumgruppe durch Halogen zu Gunsten der Anlagerungsreaktion zurückgedrängt wird (vgl. E. Müller, Angew. Chemie 61, 178 - 183, 1949).

In der praktischen Durchführung der Umsetzung werden die Reaktanten Diazoniumsalz und Acrylnitril

im Verhältnis 1:1 bis 1:8, vorzugsweise 1:2, eingesetzt. Die Reaktionstemperaturen betragen 20° bis 50° C, bevorzugt 25° - 35° C. Die Reaktionsdauer beträgt 0,5 bis 10 Stunden, vorzugsweise 1 bis 3 Stunden. Als Lösungsvermittler im wässrigen Reaktionsmedium wird bevorzugt Ethylmethylketon eingesetzt.

Bei der Abspaltung von HCl aus der Verbindung der Formel XIV werden als inerte Lösungsmittel beispielsweise aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Dioxan, Tetrahydrofuran; Nitrile wie Acetonitril, Propionitril; N,N-dialkylierte Amide wie Dimethylformamid; Dimethylsulfoxid; Ketone wie Aceton, Diethylketon, Methylethylketon und Gemische solcher Lösungsmittel untereinander verwendet. Als säurebindende Mittel dienen schwach nucleophile organische Basen, vorzugsweise Trialkylamine. Die Abspaltungsreaktion wird bei Temperaturen, die von Raumtemperatur bis zur Rückflusstemperatur des verwendeten Lösungsmittels reichen, bevorzugt von 30° bis 60° C, durchgeführt. Die Reaktionsdauer beträgt 1 bis 24 Stunden, vorzugsweise 3 bis 12 Stunden.

Die Verbindung der Formel IIb ist ein wertvolles Zwischenprodukt zur Herstellung von Fungiziden und stellt als neue Verbindung einen Teil der Erfindung dar.

Ein Teil der erwähnten, durch Reduktion in die Amine der Formeln XIa und XIb überführbaren 7-Nitrobenzfurane und 7-Nitrobenzthiophene der Formel

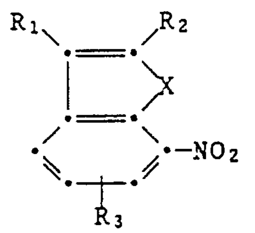

,

worin $R_1$, $R_2$, $R_3$ und X die bei der Definition der Verbindungen der Formel I angegebenen Bedeutungen haben, sind zum Teil im J. Med. Chem. 13, (6) 1102-5 (1970) und in den Ann. Chim (Roma) 47885-91 (1957) beschrieben. Die als Vorstufe der Verbindungen der Formeln XIa und XIb dienenden Nitroverbindungen können allgemein analog zu den hierin beschriebenen Methoden hergestellt werden.

N-Benzothienylmethyl-N-alkenylalkylamine als humane Antimicotica sind aus der DE-OS 3,302,814 bekannt. Die Verbindungen sind jedoch keine Benzothiophene, welche an einen substituierten oder unsubstituierten Pyrrolrest gebunden sind.

Aus der Japanischen Patentanmeldung J5-9104-374A (CA 101 191676m, 1984) ist die Herstellung von Benzofuran-Derivaten beschrieben. Die resultierenden Verbindungen sind als Zwischenprodukte für Agrochemikalien geeignet. Es sind hier jedoch keine Benzofurane erwähnt, welche an einen substituierten oder unsubstituierten Pyrrolrest gebunden sind.

Aus der EP-A 270342 sind Benzofuran- und Benzothiophen-7-carbonsäure-Derivate mit einer die reflektive Blasenkontraktion hemmenden Wirkung beschrieben.

An Pyrrolrest gebundene Benzothiophene und Benzofurane mit mikrobizider oder fungizider Wirkung sind unbekannt. Die erfindungsgemässen Benzothiophen- und Benzofuran-Derivate der Formel I sind neu.

Es wurde nun überraschend festgestellt, dass die erfindungsgemässen Verbindungen der Formel I ein sehr vorteilhaftes, die praktischen Bedürfnisse gut befriedigendes biozides Wirkungsspektrum gegen schädliche Mikroorganismen, insbesondere gegen phytopathogene Pilze und Bakterien, aufweisen. Sie besitzen sehr vorteilhafte kurative, systemische und insbesondere präventive Eigenschaften und lassen sich zum Schutz von zahlreichen Kulturpflanzen einsetzen. Mit dem Wirkstoff der Formel I können an Pflanzen oder an Pflanzenteilen (Früchte, Blüten, Laubwerk, Stengel, Knollen, Wurzeln) von unterschiedlichen Nutzkulturen die auftretenden Schädlinge eingedämmt oder vernichtet werden, wobei auch später zuwachsende Pflanzenteile vor phytopathogenen Mikroorganismen verschont bleiben.

Die erfindungsgemässen Wirkstoffe sind beispielsweise gegen die den folgenden Klassen angehörenden phytopathogenen Pilze wirksam: Ascomycetes z.B. Erysiphe, Sclerotinia, Fusarium, Monilinia, Helminthosporium; Basidiomycetes wie z.B. Puccinia, Tilletia, Rhizoctonia; sowie die der Klasse der Phycomycetes angehörenden Oomycetes wie Phytophthora. Als Pflanzenschutzmittel können die Verbindungen der Formel I mit besonders gutem Erfolg gegen wichtige Schadpilze aus der Familie der Fungi imperfecti eingesetzt werden, so z.B. gegen Cercospora, Pyricularia und vor allem gegen Botrytis. Botrytis spp (B. Cinerea, B. allii) stellen mit der Grauschimmelfäule an Reben, Erdbeeren, Aepfeln, Zwiebeln und anderen

Obst- und Gemüsesorten einen bedeutenden wirtschaftlichen Schadfaktor dar. Dabei weisen insbesondere die Verbindungen Hr. 1.1, 1.2 und 1.8 aus Tabelle 1 ein breites Wirkungsspektrum auf. Sie entfaltet nicht nur gegen Pyricularia, Botrytis und Rhizoctonia eine hervorragende fungizide Wirkung, sondern eignet sich auch zur erfolgreichen Bekämpfung von Erysiphe- und Venturia-Species. Ueberdies wirken die Verbindungen systemisch. Darüber hinaus lassen sich Verbindungen der Formel I erfolgreich zum Schutz verderblicher Waren pflanzlicher oder tierischer Herkunft einsetzen. Sie bekämpfen Schimmelpilze wie Penicillium, Aspergillus, Rhizopus, Fusarium, Helminthosporium, Nigrospora und Alternaria sowie Bakterien wie Buttersäurebakterien und Hefen wie Candida. Diese Wirksubstanzen zeigen ferner hervorragende Wirkung gegen Boden- und Samen-bürtige Pilze.

Als Pflanzenschutzmittel besitzen die Verbindungen der Formel I ein für die praktische Anwendung in der Landwirtschaft sehr günstiges Wirkungsspektrum zum Schutze von Kulturpflanzen, ohne diese durch unerwünschte Nebenwirkungen nachteilig zu beeinflussen.

Als Zielkulturen für den Pflanzenschutz gelten im Rahmen dieser Erfindung beispielsweise folgende Pflanzenarten: Getreide: (Weizen, Gerste, Roggen, Hafer, Reis, Sorghum und Verwandte); Rüben: (Zucker- und Futterrüben): Kern-, Stein- und Beerenobst: (Aepfel, Birnen, Pflaumen, Pfirsiche, Mandeln, Kirschen, Erd-, Him- und Brombeeren); Hülsenfrüchte: (Bohnen, Linsen, Erbsen, Soja): Oelkulturen: (Raps, Senf, Mohn, Oliven, Sonnenblumen, Kokos, Rizinus, Kakao, Erdnüssen); Gurkengewächse: (Kürbis, Gurken, Melonen); Fasergewächse: (Baumwolle, Flachs, Hanf, Jute); Citrusfrüchte: (Orangen, Zitronen, Pampelmusen, Mandarinen); Gemüsesorten: (Spinat, Kopfsalat, Spargel, Kohlarten, Möhren, Zwiebeln, Tomaten, Kartoffeln, Paprika); Lorbeergewächse: (Avocado, Cinnamonum, Kampfer) oder Pflanzen wie Mais, Tabak, Hüsse, Kaffee, Zuckerrohr, Tee, Weinreben, Hopfen, Bananen-und Naturkautschukgewächse sowie Zierpflanzen (Compositen).

Die Wirkstoffe der Formel I können ferner auch als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden, wobei sie sich insbesondere als Getreidebeizmittel in der Bekämpfung von Pilzorganismen, wie beispielsweise Fusarium-, Helminthosporium und Tilletia-Arten, auszeichnen.

Die Erfindung betrifft somit auch mikrobizide Mittel sowie die Verwendung der Verbindungen der Formel I zur Bekämpfung phytopathogener Mikroorganismen, insbesondere pflanzenschädigender Pilze bzw. die präventive Verhütung eines Befalls an Pflanzen und an Vorräten pflanzlicher oder tierischer Herkunft.

Unter Lager- und Vorratsgütern sollen im Rahmen vorliegender Erfindung pflanzliche und/oder tierische Naturstoffe und deren Weiterverarbeitungsprodukte verstanden werden, beispielsweise die nachfolgend aufgezählten und aus dem natürlichen Lebenszyklus herausgenommenen Pflanzen, deren Pflanzenteile (Stengel, Blätter, Knollen, Samen, Früchte, Körner), die im frisch geernteten Zustand oder in weiterverarbeitbarer Form vorliegen (vorgetrocknet, befeuchtet, zerkleinert, gemahlen, geröstet). Als Beispiele, die keinen das Anwendungsgebiet limitierenden Charakter im Rahmen dieser Erfindung besitzen, seien folgende Agrarprodukte genannt: Getreide (wie Weizen, Gerste, Roggen, Hafer, Reis, Sorghum und Verwandte); Rüben (wie Möhren, Zucker- und Futterrüben); Kern-, Stein- und Beerenobst (wie Aepfel, Birnen, Pflaumen, Pfirsiche, Mandeln, Kirschen, Erd-, Him-und Brombeeren); Hülsenfrüchte (wie Bohnen, Linsen, Erbsen, Soja); Oelkulturen (wie Raps, Senf, Mohn, Oliven, Sonnenblumen, Kokos, Rizinus, Kakao, Erdnüsse); Gurkengewächse (wie Kürbis, Gurken, Melonen); Fasergewächse (wie Baumwolle, Flachs, Hanf, Jute, Nessel); Citrusfrüchte; Gemüsesorten (wie Spinat, Salat, Spargel, Kohlarten, Zwiebeln, Tomaten, Kartoffeln, Paprika): Lorbeergewächse (wie Avocadom Cinnamonum, Kampfer) oder Mais, Tabak, Nüsse, Kaffee, Zuckerrohr, Tee, Weintrauben, Kastanien, Hopfen, Bananen, Gras und Heu.

Als Naturprodukte tierischer Herkunft seien insbesondere getrocknete Fleisch- und Fischverarbeitungsprodukte wie Trockenfleisch, Trockenfisch, Fleischkonzentrate, Knochenmehl, Fischmehl und Tiertrockenfutter genannt.

Das behandelte Vorratsgut wird durch Behandlung mit Verbindungen der Formel I nachhaltig vor dem Befall durch Schimmelpilze und andere unerwünschte Mikroorganismen geschützt. Dadurch wird die Bildung toxischer und zum Teil karzinogener Schimmelpilze (Aflatoxine und Ochratoxine) unterbunden, das Gut vor dem Verderben bewahrt und dessen Qualität für längere Zeit aufrechterhalten. Das erfindungsgemässe Verfahren kann auf alle trockenen und feuchten Vorrats- und Lagergüter angewendet werden, die gegen Mikroorganismen, wie Hefen, Bakterien und insbesondere Schimmelpilze anfällig sind.

Ein bevorzugtes Verfahren zum Aufbringen des Wirkstoffes besteht in einem Besprühen oder Benetzen des Substrats mit einer flüssigen Aufbereitung oder im Vermischen des Substrats mit einer festen Aufbereitung der Aktivsubstanz. Das beschriebene Konservierungs-Verfahren ist ein Teil der vorliegenden Erfindung.

Wirkstoffe der Formel I werden üblicherweise in Form von Zusammensetzungen verwendet und können gleichzeitig oder nacheinander mit weiteren Wirkstoffen auf die zu behandelnde Fläche, Pflanze oder Substrat gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln. Besonders vorteilhafte Zusatzstoffe sind Phospholipide.

Ein bevorzugtes Verfahren zum Aufbringen eines Wirkstoffes der Formel I bzw. eines (agro)chemischen Mittels, das mindestens einen dieser Wirkstoffe enthält, ist das Aufbringen auf das Blattwerk (Blattapplikat). Anzahl der Applikationen und Aufwandmenge richten sich dabei nach de Befallsdruck für den entsprechenden Erreger (Pilzsorte). Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z.B. in Form von Granulat (Bodenapplikation). Die Verbindungen der Formel I können aber auch auf Samenkörner aufgebracht werden (Coating), indem man die Körner entweder mit einer flüssigen Zubereitung des Wirkstoffs tränkt oder sie mit einer festen Zubereitung beschichtet. Darüber hinaus sind in besonderen Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmittel eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 20 g bis 5 kg Aktivsubstanz (AS) je ha; bevorzugt 100 g bis 2 kg AS/ha, insbesondere bei 200 g bis 600 g AS/ha.

Bei der Herstellung vom gebeizten Saatgut wird das Verhältnis Saatgut:Wirkstoff der Formel I so gewählt, dass die Aufwandmenge nach der Aussaat 0,1 g Wirkstoff/ha bis 500 g Wirkstoff/ha, vorzugsweise 0,5 g Wirkstoff/ha bis 100 g Wirkstoff/ha beträgt, d.h. die angegebene Menge Wirkstoff wird sich nach der Aussaat zusammen mit dem Saatgut auf 1 ha Ackerfläche befinden.

Die Formulierungen d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatiscshe Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl, Sonnenblumenöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht adsorptive Träger materialien z.B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände, wie z.B. Korkmehl oder Sägemehl, verwendet werden.

Als oberflächenaktive Verbindungen kommen je nach Art des zu formulierenden Wirkstoffes der Formel I nichtionosgene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren

Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-methyllaurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes. Ferner kommen auch entsprechende Phosphate wie z.B. Salze des Phosphorsäureesters eines p-Nonylphenol-(4-14-ethylenoxidaddukts in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 g Propylenglykolethergruppen enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykol einheiten.

Als Beispiele nichtionischer Tenside seien Nonylphenolpolyethoxyethanole, Ricinusölpolyglykolether, Polypropylen-Polyethylenoxidaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid. Auf dem Vorratsschutzgebiet werden die für die menschliche und tierische Ernährung unbedenklichen Zuschlagstoffe bevorzugt.

Die agrochemischen Zubereitungen enthalten in der Regel 0,1 bis 99 Gew.%, insbesondere 0,1 bis 95 Gew.%, Wirkstoff der Formel I, 99,9 bis 1 Gew.%, insbesondere 99,8 bis 5 Gew.%, eines festen oder flüssigen Zusatzstoffes und 0 bis 25 Gew.%, insbesondere 0,1 bis 25 Gew.%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige (agro)chemische Mittel sind ein Bestandteil der vorliegenden Erfindung.

Die nachfolgenden Beispiele dienen der Illustration der Erfindung, dieselbe einzuschränken (Prozente und Teile beziehen sich auf Gewicht; Temperaturen sind in Celsiusgraden angegeben).

## 1. Herstellungsbeispiele

### H.1 Herstellung von 3-Brom-7-brommethylbenzothiophen

Zu einer Lösung von 77,1 g (0,52 Mol) 7-Methylbenzothiophen in 400 ml $CCl_4$ werden bei 80°C je 0,2 g Dibenzoylperoxid, $\alpha,\alpha$-Azoisobutyronitril und 210 g (1,14 Mol) N-Bromsuccinimid in mehreren Portionen gegeben und das Reaktionsgut wird 12 Stunden lang unter Rückfluss gekocht. Anschliessend wird das Reaktionsgut auf Raumtemperatur abgekühlt, abfiltriert, das Lösungsmittel des Filtrats wird in Vakuum verdampft, der Rückstand im Gemisch Hexan: Ethylacetat = 4:1 gelöst und das Lösungsmittel nach chromatographischer Reinigung über Kieselgel wird in Vakuum verdampft. Als Rückstand werden 142 g (89

% der Theorie) des Zielprodukts isoliert.

H.2 Herstellung von 3-Brom-7-benzothiophen-carbaldehyd

Zu einer aus 14,2 g (0,61 Mol) Natrium und 800 ml abs. Ethanol unter Argonatmosphähre hergestellten Na-Ethylatlösung werden unter Rühren 68,2 g (0,74 Mol) 2-Nitropropan während 45 Minuten bei Raumtemperatur zugetropft. Zu der resultierenden weissen Suspension werden anschliessend während 1,5 Stunden bei Raumtemperatur 137 g (0,61 Mol) 3-Brom-7-brommethylbenzothiophen zugetropft. Nach eintägigem Rühren wird das Lösungsmittel aus dem Reaktionsgeisch im Vakuum verdampft, der resultierende Rückstand von 101,2 g wird in einem Gemisch Hexan:Diethylether = 1:1 gelöst und die Lösung wird über Kieselgel chromatographisch gereinigt. Nach Verdampfen des Lösungsmittels werden als Rückstand 81 g (80 % der Theorie) 3-Brom-7-benzothiophen-carbaldehyd erhalten.

H.3 Herstellung von 7-Benzothiophen-carbaldehyd

Zu einer Lösung von 10,4 g (43,15 Mmol) 3-Brom-7-benzothiophen-carbaldehyd in 130 ml Tetrahydrofuran und 5,6 ml Triethylamin werden 2 g 5 % Palladium-Kohle-Katalysator gegeben und in einer Hydrierapparatur während 45 Stunden bei 20-22 °C unter atmospherischem Druck mit Wasserstoff hydriert. Anschliessend wird der Katalysator abfiltriert und das Lösungsmittel wird aus dem Filtrat im Vakuum verdampft. Der Rückstand wird mit je 50 ml Wasser und Ethylacetat gelöst, aus der abgetrennten organischen Phase ist nach Verdampfen des Lösungsmittels im Vakuum 5,2 g (67,5 % der Theorie) 7-Benzothiophen-carbaldehyd als Rückstand erhältlich.

H.4 Herstellung von 2-Cyano-3-(benzothiophen-7-yl)-acrylsäuremethylester

Ein Gemisch von 1,0 g (6,2 Mmol) 7-Benzothiophen-carbaldehyd, 0,4 g (6,8 Mmol) Cyanessigsäuremethylester, 0,07 g Ammoniumacetat, 0,2 g Essigsäure, 1 g Molekularsieb (3Å) und 10 ml Toluol werden während 4 Stunden unter Rückfluss gekocht anschliessend wird das Reaktionsgut abgekühlt und mit 20 ml Ethylacetat und mit 20 ml 10 % NaCl-Lösung vermischt. Die abgetrennte organische Phase wird mit $MgSO_4$ getrocknet, filtriert und das Lösungsmittel wird im Vakuum verdampft. Als Rückstand wird 1,5 g 2-Cyano-3-(benzothiophen-7-yl)-acrylsäuremethylester als Rohprodukt erhalten.

H.5 Herstellung von 2-Cyano-3-(Brombenzothiophen-7-yl)-acrylsäuremethylester

1,2 g 3-Brom-7-benzothiophen-carbaldehyd wird analog zum Beispiel H.4 mit Cyanessigsäuremethylester zum 2-Cyano-3-(Brombenzothiophen-7-yl)-acrylsäuremethylester umgesetzt.

H.6 Herstellung von 3-(Benzthiophen-7-yl)-4-cyanopyrrol (Verbindung Nr. 1)

Zu einer bei 5 °C vorgelegten Mischung von 2,2 g (9,05 Mmol) 2-Cyano-3-(benzothiophen-7-yl)-acrylsäuremethylester und 1,8 g (9,05 Mmol) p-Toluolsulfonylmethylisocyanid ("Tosmic") in 13 ml Methanol wird bei 5 °C die Lösung von 2 g (10,8 Mmol) Na-Methylat, gelöst in 5 ml Methanol gegeben. Nach zweistündigem Rühren bei Raumtemperatur werden weitere 2 g Na-Methylat, gelöst in 5 ml Methanol zum Reaktionsgemisch gegeben. Anschliessend wird das Reaktionsgemisch auf 200 ml Wasser ausgegossen und 30 Min. lang damit verrührt. Das als Suspension ausgefallene Produkt wird abfiltriert und in 20 ml Ethylacetat gelöst. Die Mutterlauge wird mit 20 ml Ethylacetat extrahiert, die vereinigten Ethylacetat-Lösungen werden mit gesättigter NaCl-Lösung neutralgewaschen, über $MgSO_4$ getrocknet und das Lösungsmittel wird im Vakuum verdampft. Das als Rückstand resultierende Rohprodukt wiegt 1,8 g (90 % der Theorie). Das im Gemisch Hexan:Ethylacetat = 2:1 aufgelöste Produkt wird über Kieselgel chromatographisch gereinigt. Nach Verdampfen des Lösungsmittels wird 1,4 g (70 % der Theorie) seines 2-Cyano-3-(-Brombenzothiophen-7-yl)-acrylsäuremethylester erhalten. Smp.: 138-139 °C.

H.7 Herstellung von 3-(3-Brombenzothiophen-7-yl)-4-cyanopyrrol (Verbindung Nr. 2)

19 g (59 Mmol) 2-Cyano-3-(Brombenzothiophen-7-yl)-acrylsäuremethylester werden analog zum Beispiel H.6 mit "Tosmic" zum 3-(3-Brombenzothiophen-7-yl)-4-cyanopyrrol vom Smp.: 130-134 °C umgesetzt. Reinausbeute 83 % der Theorie.

Die Tabelle 1 enthält die auf analoge Weise herstellbaren Verbindungen bzw. auch deren Derivate, welche nach der vorangehenden Beschreibung herstellbar sind.

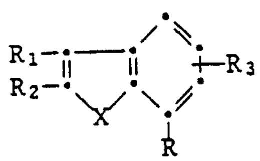

$R_3$ = H,

R =

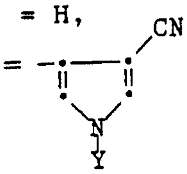

Tabelle 1

| Verb. No. | X | $R_1$ | $R_2$ | Y | Physikalische Konstanten |
|---|---|---|---|---|---|
| 1 | S | H | H | H | Smp. = 138-139 °C |
| 2 | S | Br | H | H | Smp. = 130-134 °C |
| 3 | S | H | $CF_3$ | H | |
| 4 | S | H | $CH_3$ | H | |
| 5 | S | $CH_2Br$ | H | H | |
| 6 | O | H | $CH_3$ | H | Smp. = 141-144 °C |
| 7 | O | $CH_2Br$ | H | H | |
| 8 | O | H | H | H | Smp. = 148-149 °C |
| 9 | O | Br | H | H | |
| 10 | S | H | H | CHO | |
| 11 | S | H | H | $COCH_3$ | |
| 12 | S | $CH_3$ | H | $COCH_2CH_3$ | |
| 13 | S | H | H | $SCCl_2F$ | |
| 14 | S | Br | H | $COCH_3$ | |
| 15 | O | H | H | CHO | |
| 16 | O | $CH_3$ | H | $COCH_2CH_3$ | |
| 17 | O | H | H | $SCCl_2F$ | |
| 18 | O | Br | H | $COCH_3$ | |

2. Formulierungsbeispiele

| 2.1. Emulsion-Konzentrate | a) | b) | c) |
|---|---|---|---|
| Wirkstoff aus Tabelle 1 | 25% | 40% | 50% |
| Ca-Dodecylbenzolsulfonat | 5% | 8% | 6% |
| Ricinusöl-polyäthylenglykoläther (36 Mol Aethylenoxid) | 5% | - | - |
| Tributylphenol-polyäthylenglykoläther (30 Mol Aethylenoxid) | - | 12% | 4% |
| Cyclohexanon | - | 15% | 20% |
| Xylolgemisch | 65% | 25% | 20% |

Aus solchen Konzentraten können durch Verdünnen mit Wasser Emulsionen jeder gewünschten

Konzentration hergestellt werden.

| 2.2. Lösungen | a) | b) | c) | d) |
|---|---|---|---|---|
| Wirkstoff aus Tabelle 1 | 80% | 10% | 5% | 95% |
| Aethylenglykol-monomethyläther | 20% | - | - | - |
| Polyäthylenglykol M G 400 | - | 70% | - | - |
| N-Methyl-2-pyrrolidon | - | 20% | - | - |
| Epoxydiertes Kokosnussöl | - | - | 1% | 5% |
| Benzin (Siedegrenzen 160-190 °C) | - | - | 94% | - |
| (MG = Molekulargewicht) | | | | |

| 2.3. Granulate | a) | b) |
|---|---|---|
| Wirkstoff aus Tabelle 1 | 5% | 10% |
| Kaolin | 94% | |
| Hochdisperse Kieselsäure | 1% | - |
| Attapulgit | - | 90% |

Der Wirkstoff wird in Methylenchlorid gelöst. auf den Träger aufgesprüht und das Lösungsmittel anschliessend im Vakuum abgedampft.

| 2.4. Stäubemittel | a) | b) |
|---|---|---|
| Wirkstoff aus Tabelle 1 | 2% | 5% |
| Hochdisperse Kieselsäure | 1% | 5% |
| Talkum | 97% | - |
| Kaolin | - | 90% |

Durch inniges Vermischen der Trägerstoffe mit dem Wirkstoff erhält man gebrauchsfertige Stäubemittel.

| 2.5. Spritzpulver | a) | b) | c) |
|---|---|---|---|
| Wirkstoff aus Tabelle 1 | 25% | 50% | 75% |
| Na-Ligninsulfonat | 5% | 5% | - |
| Na-Laurylsulfat | 3% | - | 5% |
| Na-Diisobutylnaphthalinsulfonat | - | 6% | 10% |
| Octylphenolpolyäthylenglykoläther (7-8 Mol Aethylenoxid | - | 2% | - |
| Hochdisperse Kieselsäure | 5% | 10% | 10% |
| Kaolin | 62% | 27% | - |

Der Wirkstoff wird mit den Zusatzstoffen gut vermischt und in einer geeigneten Mühle vermahlen. Man erhält Spritzpulver, die sich mit Wasser zu Suspensionen jeder gewünschten Konzentration verdünnen lassen.

| 2.6. Emulsions-Konzentrat | |
|---|---|
| Wirkstoff aus Tabelle 1 | 10% |
| Octylphenolpolyäthylenglykoläther (4-5 Mol Aethylenoxid) | 3% |
| Ca-Dodecylbenzolsulfonat | 3% |
| Ricinusölpolyglykoläther (35 Mol Aethylenoxid) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| 2.7. Stäubemittel | a) | b) |
|---|---|---|
| Wirkstoff aus Tabelle 1 | 5% | 8% |
| Talkum | 95% | - |
| Kaolin | - | 92% |

Man erhält anwendungsfertige Stäubemittel, indem der Wirkstoff mit dem Träger vermischt auf einer geeigneten Mühle vermahlen wird.

| 2.8. Extruder Granulat | |
|---|---|
| Wirkstoff aus Tabelle 1 | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

| 2.9. Umhüllungs-Granulat | |
|---|---|
| Wirkstoff aus Tabelle 1 | 3% |
| Polyäthylenglykol M G 200 | 3% |
| Kaolin | 94% |
| (MG = Molekulargewicht) | |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| 2.10. Suspensions-Konzentrat | |
|---|---|
| Wirkstoff aus Tabelle 1 | 40% |
| Aethylenglykol | 10% |
| Nonylphenolpolyäthylenglykoläther (15 Mol Aethylenoxid) | 6% |
| Na-Ligninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

3. Biologische Beispiele:

Beispiel 3.1.: Wirkung gegen Puccinia graminis auf Weizen

a) Residual-protektive Wirkung

Weizenpflanzen werden 6 Tage nach der Aussaat mit einer aus Spritzpulver des Wirkstoffes hergestellten Spritzbrühe (0,02 % Aktivsubstanz) besprüht. Nach 24 Stunden werden die behandelten Pflanzen mit einer Ureidosporensuspension des Pilzes infiziert. Nach einer Inkubation während 48 Stunden bei 95 - 100 % relativer Luftfeuchtigkeit und ca. 20° C werden die infizierten Pflanzen in einem Gewächshaus bei ca. 22° C aufgestellt. Die Beurteilung der Rostpustelnentwicklung erfolgt 12 Tage nach der Infektion.

b) Systemische Wirkung

Zu Weizenpflanzen wird 5 Tage nach der Aussaat eine aus Spritzpulver des Wirkstoffes hergestellte Spritzbrühe gegossen (0,006 % Aktivsubstanz bezogen auf das Bodenvolumen). Nach 48 Stunden werden die behandelten Pflanzen mit einer Ureidosporensuspension des Pilzes infiziert. Nach einer Inkubation während 48 Stunden bei 95 - 100 % relativer Luftfeuchtigkeit und ca. 20° C werden die infizierten Pflanzen in einem Gewächshaus bei ca. 22° C aufgestellt. Die Beurteilung der Rostpustelnentwicklung erfolgt 12 Tage nach der Infektion.

Verbindungen aus der Tabelle 1 zeigten gegen Puccinia-Pilze eine gute Wirkung. Unbehandelte aber infizierte Kontrollpflanzen zeigten eine Puccinia-Befall von 100 %. Unter anderen hemmten die Verbindungen Nr. 1, 2 und 8 den Puccinia-Befall auf 0 bis 5 %.

Beispiel 3.2.: Wirkung gegen Cercospora arachidicola auf Erdnusspflanzen

Residual-protektive Wirkung

10 - 15 cm hohe Erdnusspflanzen werden mit einer aus Spritzpulver der Wirksubstanz hergestellten Spritzbrühe (0,006 % Aktivsubstanz besprüht und 48 Stunden später mit einer Konidiensuspension des Pilzes infiziert. Die infizierten Pflanzen werden während 72 Stunden bei ca. 21° C und hoher Luftfeuchtigkeit inkubiert und anschliessend bis zum Auftreten der typischen Blattflecken in einem Gewächshaus aufgestellt. Die Beurteilung der fungiziden Wirkung erfolgt 12 Tage nach der Jnfektion basierend auf Anzahl und Grösse der auftretenden Flecken.

Im Vergleich zu unbehandelten, aber infizierten Kontrollpflanzen (Anzahl und Grösse der Flecken = 100 %), zeigten Erdnusspflanzen, die mit Wirkstoffen aus der Tabelle 1 behandelt wurden, einen stark reduzierten Cercospora-Befall. So verhinderten die Verbindungen Nr. 1, 2 und 8 in obigen Versuchen das Auftreten von Flecken fast vollständig.

Beispiel 3.3.: Wirkung gegen Erysiphe graminis auf Gerste

Residual-protektive Wirkung

Ca. 8 cm hohe Gerstenpflanzen werden mit einer aus Spritzpulver des Wirkstoffes hergestellten Spritzbrühe (0,02 % Aktivsubstanz) besprüht. Nach 3 - 4 Stunden werden die behandelten Pflanzen mit

Konidien des Pilzes bestäubt. Die infizierten Gerstenpflanzen werden in einem Gewächshaus bei ca. 22°C aufgestellt und der Pilzbefall nach 10 Tagen beurteilt. Die Verbindungen Nr. 1 und 2aus der Tabelle 1 waren gut wirksam gegen Erysiphe-Befall von Gerste.

Beispiel 3.4.: Residual-protektive Wirkung gegen Venturia inaequalis auf Apfeltrieben

Apfelstecklinge mit 10 - 20 cm langen Frischtrieben werden mit einer aus Spritzpulver des Wirkstoffes hergestellten Spritzbrühe (0,06 % Aktivsubstanz) besprüht. Nach 24 Stunden werden die behandelten Pflanzen mit einer Konidiensuspension des Pilzes infiziert. Die Pflanzen werden dann während 5 Tagen bei 90 - 100 % relativer Luftfeuchtigkeit inkubiert und während 10 weiteren Tagen in einem Gewächshaus bei 20 - 24°C aufgestellt. Der Schorbefall wird 1 - 5 Tage nach der Jnfektion beurteilt. Wirkstoffe aus der Tabelle 1 zeigten gute Wirksamkeit gegen Venturia auf Apfeltrieben. Verbindungen Nr. 1, 2 und 8 hemmten den Krankheitsbefall auf weniger als 10 %. Unbehandelte aber infizierte Triebe zeigten einen 100 %igen Venturia-Befall

Beispiel 3.5.: Wirkung gegen Botrytis cinerea auf Bohnen

Residual protektive Wirkung

Ca. 10 cm hohe Bohnen-Pflanzen werden mit einer aus Spritzpulver des Wirkstoffes hergestellten Spritzbrühe (0,02 % Aktivsubstanz) besprüht. Nach 48 Stunden werden die behandelten Pflanzen mit einer Konidiensuspension des Pilzes infiziert. Nach einer Inkubation der infizierten Pflanzen während 3 Tagen bei 95 - 100 % relativer Luftfeuchtigkeit und 21°C erfolgt die Beurteilung des Pilzbefalls. Die Verbindungen aus der Tabelle 1 hemmten in vielen Fällen die Pilzinfektion vollständig. Bei einer Konzentration von 0.02 % erwiesen sich z.B. die Verbindungen Nr. 1, 2 und 8 als voll wirksam (kein sichtbarer Krankheitsbefall) . Der Befall unbehandelter aber infizierter Bohnenpflanzen betrug 100 %.

Beispiel 3.6.: Wirkung gegen Botrytis cinerea an Apfelfrüchten

Künstlich verletzte Aepfel werden behandelt, indem eine aus Spritzpulver der Wirksubstanz hergestellte Spritzbrühe auf die Verletzungsstellen aufgetropft wird. Die behandelten Früchte werden anschliessend mit einer Sporensuspension von Botrytis cinerea inokuliert und während einer Woche bei einer hohen Luftfeuchtigkeit und ca. 20°C inkubiert.

Bei der Auswertung werden die angefaulten Verletzungsstellen gezählt und daraus die fungizide Wirkung der Testsubstanz abgeleitet. Verbindungen aus Tabelle 1 waren gut wirksam gegen Botrytis-Befall von Apfelfrüchten. Unter anderen hemmten die Verbindungen Nr. 1, 2 und 8 den Pilzbefall im Vergleich zu unbehandelten Kontrollfrüchten (mit 100 % Befall) fast vollständig (Krankheitsbefall 0 - 5 %).

**Ansprüche**

1. Benzothiophen- und Benzofuran-Derivate der Formel I

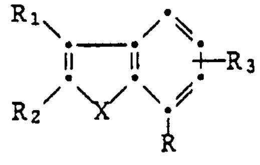

(I),

worin X Sauerstoff oder Schwefel, $R_1$ Wasserstoff, Halogen, $C_1$-$C_4$-Alkyl, $C_1$-$C_4$-Haloalkyl, $R_2$ Wasserstoff, Halogen, $C_1$-$C_4$-Alkyl, $C_1$-$C_4$-Haloalkyl, $R_3$ Wasserstoff, $C_1$-$C_4$-Alkyl, Halogen und R einen 4-Cyano-3-pyrrolrest bedeutet, dessen N-Atom einen der folgenden Substituenten trägt:

A: Wasserstoff oder CO-$R_4$, wobei $R_4$ unsubstituiertes $C_1$-$C_6$-Alkyl oder durch Halogen oder $C_1$-$C_3$-

Alkoxy substituiertes $C_1$-$C_6$-Alkyl; $C_3$-$C_6$-Alkenyl, $C_3$-$C_6$-Alkinyl, unsubstituiertes $C_1$-$C_6$-Alkoxy oder durch Halogen oder $C_1$-$C_3$-Alkoxy substituiertes $C_1$-$C_6$-Alkoxy; $C_3$-$C_6$-Alkenyloxy, $C_3$-$C_6$-Cycloalkyl oder Tetrahydrofur-2-yl darstellt;

B: S-$R_5$, worin $R_5$ $C_1$-$C_3$-Haloalkyl bedeutet;

C: CH(Y)$R_6$, wobei $R_6$ für Wasserstoff oder $C_1$-$C_8$-Haloalkyl und Y für Hydroxy, Halogen oder OC(O)-$R_7$ stehen, worin $R_7$ $C_1$-$C_8$-Alkyl, $C_1$-$C_8$-Haloalkyl, $C_2$-$C_6$-Alkenyl, 2-Tetrahydrofuryl, 2-Tetrahydropyranyl oder $C_1$-$C_6$-Alkoxycarbonyl darstellen;

D: $CH_2$-Z, wobei Z für eine der Gruppen

a) 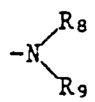 oder b) 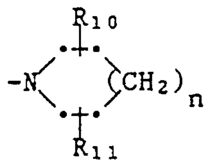 oder c) 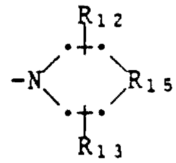

steht, worin $R_8$ und $R_9$ unabhängig voneinander Wasserstoff, unsubstituiertes oder durch Cyano, $C_1$-$C_6$-Alkoxycarbonyl substituiertes $C_1$-$C_6$-Alkyl; $C_3$-$C_6$-Alkenyl, $C_3$-$C_6$-Alkinyl, $C_3$-$C_7$-Cycloalkyl, unsubstituiertes oder durch Halogen, $C_1$-$C_6$-Alkyl, $C_1$-$C_6$-Haloalkyl und/oder $C_1$-$C_6$-Alkoxy substituiertes Phenyl bedeuten, mit der Massgabe, dass nur $R_8$ oder $R_9$ Wasserstoff sein kann; $R_{10}$ und $R_{11}$ unabhängig voneinander für Wasserstoff, $C_1$-$C_6$-Alkyl, $C_1$-$C_6$-Alkoxycarbonyl darstellen; $R_{12}$ und $R_{13}$ unabhängig voneinander für Wasserstoff, $C_1$-$C_6$-Alkyl oder $C_1$-$C_6$-Alkoxycarbonyl stehen; und $R_{15}$ Sauerstoff, Schwefel, $>C=O$ oder $>N$-$R_{14}$ bedeutet, worin $R_{14}$ Wasserstoff, $C_1$-$C_6$-Alkyl, Formyl, $C_1$-$C_6$-Alkanoyl oder $C_1$-$C_6$-Alkoxycarbonyl darstellt; und n für eine der Zahlen 0 oder 1 steht.

2. Verbindungen gemäss Anspruch 1, bei denen der Pyrrolrest am N-Atom einen der folgenden Reste trägt:
Wasserstoff oder CO-$R_4$, wobei $R_4$ unsubstituiertes $C_1$-$C_6$-Alkyl oder durch Halogen oder $C_1$-$C_3$-Alkoxy substituiertes $C_1$-$C_6$-Alkyl; $C_3$-$C_6$-Alkenyl, $C_3$-$C_6$-Alkinyl, unsubstituiertes $C_1$-$C_6$-Alkoxy oder durch Halogen oder $C_1$-$C_3$-Alkoxy substituiertes $C_1$-$C_6$-Alkoxy; $C_3$-$C_6$-Alkenyloxy, $C_3$-$C_6$-Cycloalkyl oder Tetrahydrofur-2-yl darstellt.

3. Verbindungen gemäss Anspruch 2, bei denen das N-Atom des Pyrrols einen der folgenden Resten trägt:
Wasserstoff oder $COR_4$, wobei $R_4$ unsubstituiertes $C_1$-$C_4$-Alkyl oder durch Chlor, Brom oder $C_1$-$C_3$-Alkoxy substituiertes $C_1$-$C_6$-Alkyl; $C_3$-$C_4$-Alkenyl, $C_3$-$C_4$-Alkinyl, unsubstituiertes $C_1$-$C_4$-Alkoxy, oder durch Chlor, Brom oder $C_1$-$C_3$-Alkoxy substituiertes $C_1$-$C_4$-Alkoxy; $C_3$-$C_4$-Alkenyloxy, $C_3$-$C_6$-Cycloalkyl oder Tetrahydrofur-2-yl, darstellt.

4. Verbindungen gemäss Anspruch 3, bei denen der Pyrrolrest am N-Atom ein Wasserstoffatom trägt.

5. Verbindungen gemäss Anspruch 4, bei denen $R_1$ Wasserstoff, Halogen, $C_1$-$C_2$-Alkyl oder $C_1$-$C_2$-Haloalkyl, $R_2$ Wasserstoff oder Halogen und $R_3$ Wasserstoff bedeuten.

6. Verbindungen gemäss Anspruch 5, bei denen $R_1$ Wasserstoff oder Halogen und $R_2$ Wasserstoff bedeuten.

7. Das 3-(Brombenzothiophen-7-yl)-4-cyanopyrrol gemäss Anspruch 6.

8. Das 3-(Benzothiophen-7-yl)-4-cyanopyrrol gemäss Anspruch 6.

9. Verfahren zur Herstellung der Verbindungen der Formel I in Anspruch 1, dadurch gekennzeichnet, dass

a) die Verbindungen der Formeln IIa oder IIb mit p-Toluolsulfonylmethylisocyanid in einem organischen Lösungsmittel in Gegenwart einer Base bei Temperaturen von -30° bis 120° C, vorzugsweise bei -30° bis 50° C, zu einer Verbindung der Formel Ia umgesetzt werden:

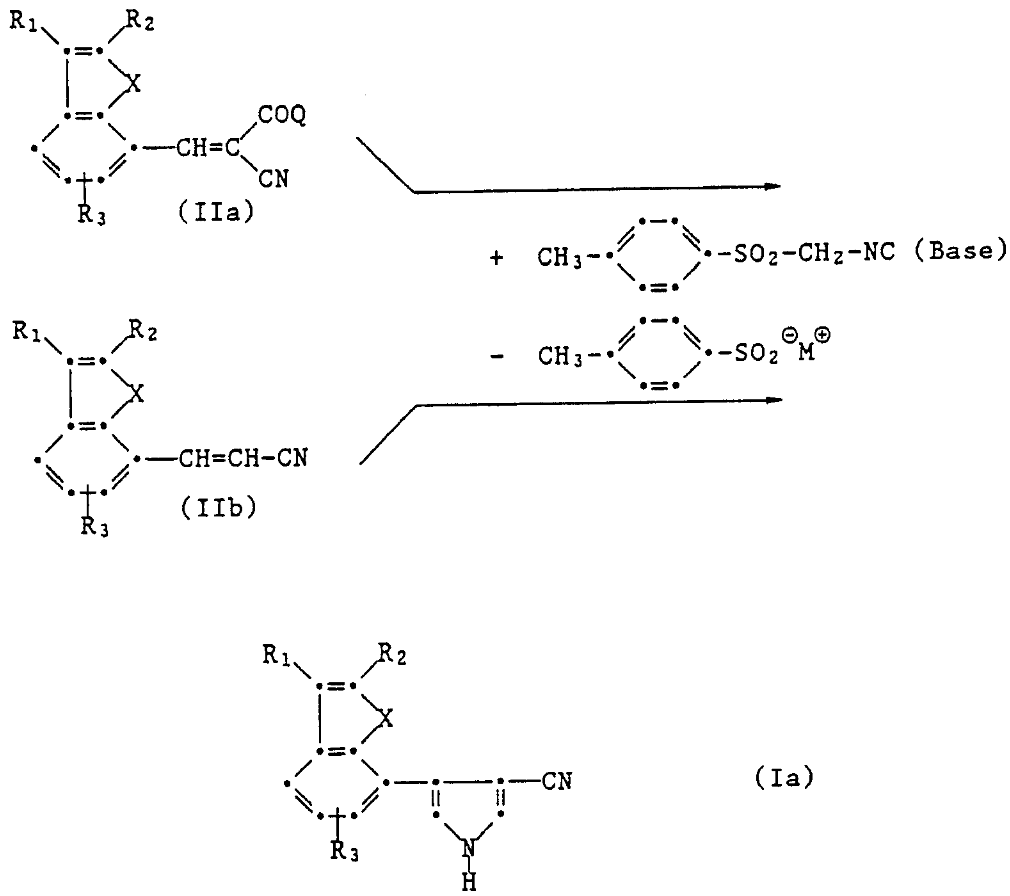


(IIa)

(IIb)

(Ia)

wobei $M^{\oplus}$ ein Alkali- oder Erdalkaliion und Q $OR_{16}$ oder $NH_2$ bedeuten und $R_{16}$ für Wasserstoff oder $C_1$-$C_4$-Alkyl steht und

b) anschliessend die Verbindung der Formel Ia in Gegenwart eines säurebindenden Mittels und gegebenenfalls eines Katalysators in einem organischen Lösungsmittel bei Temperaturen von -25° bis 100° C, vorzugsweise von -10° bis 75° C, acyliert wird:

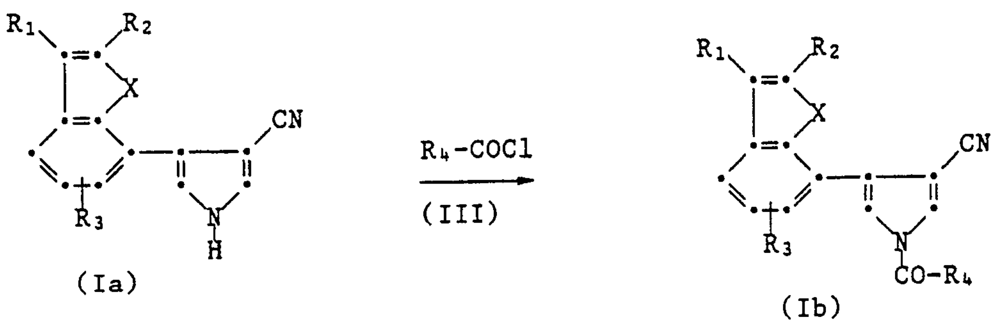


(Ia)

(Ib)

wobei $R_4$ die unter Formel I in Anspruch 1 angegebenen Bedeutungen hat, oder

c) die Verbindung der Formel Ia mit einem reaktionsfähigen Säurederivat einer Sulfensäure der Formel IV

$R_5$S-OH (IV)

in Gegenwart eines säurebindenden Mittels gegebenenfalls in einem organischen Lösungsmittel bei Temperaturen von -30° bis 100° C, vorzugsweise -10° bis 20° C, umgesetzt wird oder

d) die Verbindung der Formel Ia mit einem Aldehyd der Formel V zu einem Hydroxyderivat der Formel Ic bei Temperaturen von 0° bis 200° C, vorzugsweise 0° bis 160° C, umgesetzt wird:

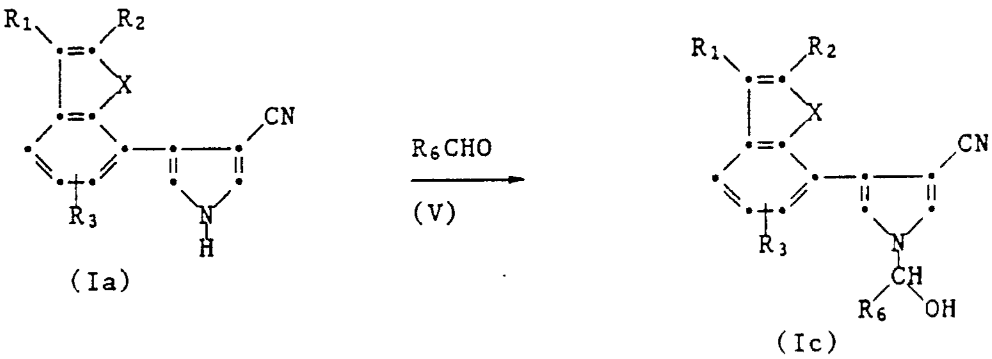


(Ia) (Ic)

und anschliessend an der Verbindung Ic die OH-Gruppe gegen einen anderen Rest Y ausgetauscht wird, indem eine Verbindung der Formel Ic entweder mit einer Säure der Formel VI
$R_7COOH$ (VI)
oder mit einem Säurehalogenid oder Säureanhydrid davon zu dem Acyloxyderivat der Formel Id

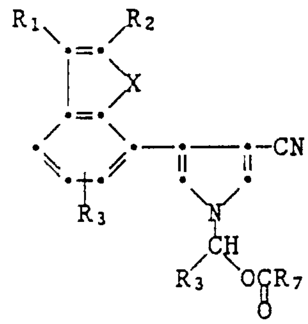

(Id)

in einem inerten Lösungsmittel umgesetzt wird,
oder zuerst die OH-Gruppe der Verbindung der Formel Ic gegen ein Halogenatom ausgetauscht wird, was eine Verbindung der Formel Ie

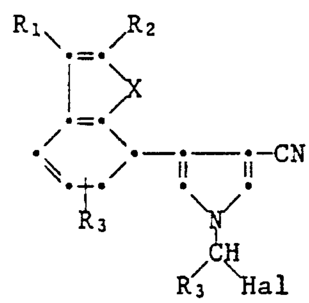

(Ie)

ergibt, und diese dann weiter durch Reaktion mit einem Salz der Formel VII
$R_7\text{-}COO^{\ominus} M'^{\oplus}$ (VII)
in einem inerten Lösungsmittel bei Temperaturen von 0° bis 150°C, vorzugsweise 20° bis 80°C, in eine Verbindung der Formel Id übergeführt wird, wobei die Reste $R_6$ und $R_7$ die unter Formel I in Anspruch 1 an gegebenen Bedeutungen besitzt, Hal für Halogen, vorzugsweise Chlor und Brom, steht und $M'^{\oplus}$ ein Metallkation, vorzugsweise $Na^{\oplus}$, $K^{\oplus}$ $Mg^{\oplus\oplus}$ oder $Ca^{\oplus\oplus}$, darstellen,
oder

e) die Verbindung der Formel Ia mit einer Verbindung der Formel VIII
H-Z (VIII)
entweder in einem protischen Lösungsmittel bei Temperaturen von 0° bis 120°C, vorzugsweise 20°C bis zur Rückflusstemperatur des verwendeten Lösungsmittels, in Gegenwart eines basischen oder sauren Reaktionskatalysators mit Formaldehyd; oder in einem aprotischen Lösungsmittel in Gegenwart eines basischen Reaktionskatalysators bei Temperaturen von 0° bis 120°C, vorzugsweise 20° bis 80°C, mit 1,3,5-Trioxan oder Paraformaldehyd umgesetzt wird, wobei der Rest Z die unter Formel I in Anspruch 1

angegebenen Bedeutungen besitzt.

10. Verbindungen der Formel IIa

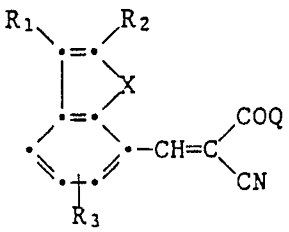

(IIa),

worin $R_1$, $R_2$, $R_3$ und X die im Anspruch 1 angegebenen Bedeutungen haben und Q für $OR_{16}$ oder $NH_2$ steht und $R_{16}$ Wasserstoff oder $C_1$-$C_4$-Alkyl bedeutet.

11. Verbindungen der Formel IIb

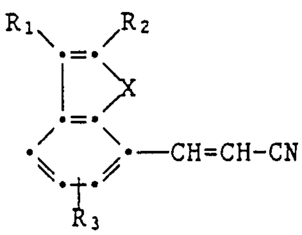

(IIb),

worin $R_1$, $R_2$, $R_3$ und X die im Anspruch 1 angegebenen Bedeutungen haben.

12. Mikrobizides Mittel zur Bekämpfung oder Verhütung eines Befalls von lebenden Pflanzen und/oder zur Konservierung von verderblichem Lagergut pflanzlicher oder tierischer Herkunft, dadurch gekennzeichnet, dass es als mindestens eine aktive Komponente eine der in Anspruch 1 definierten Verbindungen enthält.

13. Mittel gemäss Anspruch 12, dadurch gekennzeichnet, dass es als aktive Komponente mindestens eine der in den Ansprüchen 2 bis 8 definierten Verbindungen enthält.

14. Mittel nach einem der Ansprüche 12 oder 13, dadurch gekennzeichnet, dass es 0,1 bis 99 % eines Wirkstoffs der Formel I, 99,9 bis 1 % eines festen oder flüssigen Zusatzstoffes und 0 bis 25 % eines Tensides enthält.

15. Mittel nach Anspruch 14, dadurch gekennzeichnet, dass es 0,1 bis 95 % eines Wirkstoffes der Formel I 99,8 bis 5 % eines festen oder flüssigen Zusatzstoffes und 0,1 bis 25 % eines Tensides enthält.

16. Verfahren zur Herstellung eines in den Ansprüchen 12 bis 15 definierten (agro)chemischen Mittels, dadurch gekennzeichnet, dass man mindestens eine Verbindung der Formel I gemäss einem der Ansprüche 1 bis 8 mit geeigneten festen oder flüssigen Zusatzstoffen und Tensiden innig vermischt.

17. Verfahren zur Bekämpfung oder Verhütung eines Befalls von Kulturpflanzen durch phytopathogene Mikroorganismen, dadurch gekennzeichnet, dass man eine Verbindung der Formel I gemäss einem der Ansprüche 1 bis 8 auf die Pflanze, Pflanzenteile oder deren Standort appliziert.

18. Verfahren zur Beizung von Saatgut und Pflanzenstecklingen zum Schutz gegen den Befall durch Pilzorganismen, dadurch gekennzeichnet, dass man eine Verbindung der Formel I gemäss einem der Ansprüche 1 bis 8 auf das Saatgut oder die Pflanzenstecklinge appliziert.

19. Verfahren zur Konservierung bzw. zum Schutz von Vorrats- oder Lagergut pflanzlicher und/oder tierischer Herkunft vor schädlichen Mikroorganismen durch Behandlung des Gutes mit mindestens einer mikrobizid wirksamen Menge einer Verbindung der Formel I gemäss einem der Ansprüche 1 bis 8.

20. Verwendung von Verbindungen der Formel I gemäss Anspruch 1 zur Bekämpfung und/oder zur präventiven Verhütung eines Befalls von Mikroorganismen.

21. Verwendung nach Anspruch 20 von Verbindungen der Formel I gemäss einem der Ansprüche 2 bis 8.

22. Verwendung gemäss einem der Ansprüche 20 oder 21, dadurch gekennzeichnet, dass es sich bei den Mikroorganismen um phytopathogene Pilze handelt.

23. Verwendung gemäss Anspruch 22 gegen Pilze aus den Klassen Ascomycetes, Basidiomycetes oder Fungi imperfecti.

24. Verwendung gemäss Anspruch 23 gegen Botrytis-Pilze.

Patentansprüche für folgenden Vertragsstaat: ES

1. Verfahren zur Herstellung von Benzothiophen- und Benzofuran-Derivaten der Formel I

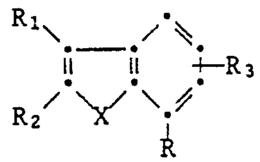 (I),

worin X Sauerstoff oder Schwefel, $R_1$ Wasserstoff, Halogen, $C_1$-$C_4$-Alkyl, $C_1$-$C_4$-Haloalkyl, $R_2$ Wasserstoff, Halogen, $C_1$-$C_4$-Alkyl, $C_1$-$C_4$-Haloalkyl, $R_3$ Wasserstoff, $C_1$-$C_4$-Alkyl, Halogen und R einen 4-Cyano-3-pyrrolrest bedeutet, dessen N-Atom einen der folgenden Substituenten trägt:
A: Wasserstoff oder CO-$R_4$, wobei $R_4$ unsubstituiertes $C_1$-$C_6$-Alkyl od durch Halogen oder $C_1$-$C_3$-Alkoxy substituiertes $C_1$-$C_6$-Alkyl; $C_3$-$C_6$-Alkenyl, $C_3$-$C_6$-Alkinyl, unsubstituiertes $C_1$-$C_6$-Alkoxy oder durch Halogen oder $C_1$-$C_3$-Alkoxy substituiertes $C_1$-$C_6$-Alkoxy; $C_3$-$C_6$-Alkenyloxy, $C_3$-$C_6$-Cycloalkyl oder Tetrahydrofur-2-yl darstellt;
B: S-$R_5$, worin $R_5$ $C_1$-$C_3$-Haloalkyl bedeutet;
C: $CH(Y)R_6$, wobei $R_6$ für Wasserstoff oder $C_1$-$C_8$-Haloalkyl und Y für Hydroxy, Halogen oder $OC(O)R_7$ stehen, worin $R_7$ $C_1$-$C_8$-Alkyl, $C_1$-$C_8$-Haloalkyl, $C_2$-$C_6$-Alkenyl, 2-Tetrahydrofuryl, 2-Tetrahydropyranyl oder $C_1$-$C_6$-Alkoxycarbonyl darstellen;
D: $CH_2$-Z, wobei Z für eine der Gruppen

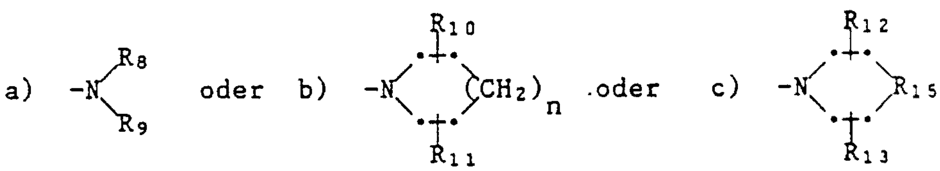

steht, worin $R_8$ und $R_9$ unabhängig voneinander Wasserstoff, unsubstituiertes oder durch Cyano, $C_1$-$C_6$-Alkoxycarbonyl substituiertes $C_1$-$C_6$-Alkyl; $C_3$-$C_6$-Alkenyl, $C_3$-$C_6$-Alkinyl, $C_3$-$C_7$-Cycloalkyl, unsubstituiertes oder durch Halogen, $C_1$-$C_6$-Alkyl, $C_1$-$C_6$-Haloalkyl und/oder $C_1$-$C_6$-Alkoxy substituiertes Phenyl bedeuten, mit der Massgabe, dass nur $R_8$ oder $R_9$ Wasserstoff sein kann; $R_{10}$ und $R_{11}$ unabhängig voneinander für Wasserstoff, $C_1$-$C_6$-Alkyl, $C_1$-$C_6$-Alkoxycarbonyl darstellen; $R_{12}$ und $R_{13}$ unabhängig voneinander für Wasserstoff, $C_1$-$C_6$-Alkyl oder $C_1$-$C_6$-Alkoxycarbonyl stehen; und $R_{15}$ Sauerstoff, Schwefel, $>C=O$ oder $>N-R_{14}$ bedeutet, worin $R_{14}$ Wasserstoff, $C_1$-$C_6$-Alkyl, Formyl, $C_1$-$C_6$-Alkanoyl oder $C_1$-$C_6$-Alkoxycarbonyl darstellt; und n für eine der Zahlen 0 oder 1 steht,
dadurch gekennzeichnet, dass

a) die Verbindungen der Formeln IIa oder IIb mit p-Toluolsulfonylmethylisocyanid in einem organischen Lösungsmittel in Gegenwart einer Base bei Temperaturen von -30° bis 120°C, vorzugsweise bei -30° bis 50°C, zu Verbindung der Formel Ia umgesetzt werden:

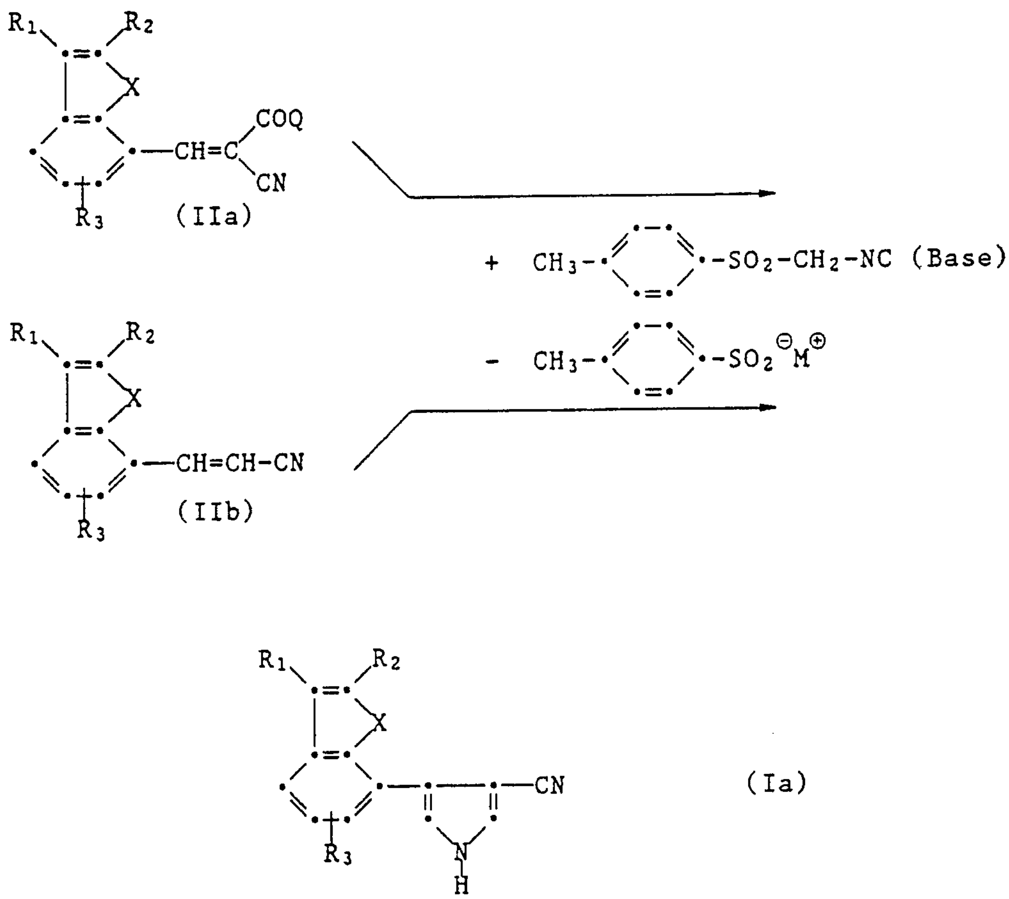


(IIa)

(IIb)

(Ia)

wobei $M^{\oplus}$ ein Alkali- oder Erdalkaliion und Q $OR_{16}$ oder $NH_2$ bedeuten und $R_{16}$ für Wasserstoff oder $C_1$-$C_4$-Alkyl steht und

b) anschliessend die Verbindung der Formel Ia in Gegenwart eines säurebindenden Mittels und gegebenenfalls eines Katalysators in einem organischen Lösungsmittel bei Temperaturen von -25° bis 100° C, vorzugsweise von -10° bis 75° C, acyliert wird:

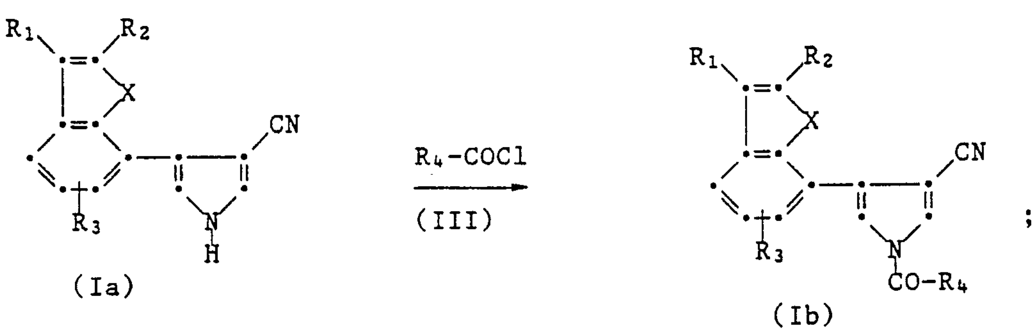


(Ia) (Ib) ;

c) die Verbindung der Formel Ia mit einem reaktionsfähigen Säurederivat einer Sulfensäure der Formel IV

$R_5$S-OH (IV)

in Gegenwart eines säurebindenden Mittels gegebenenfalls in einem organischen Lösungsmittel bei Temperaturen von -30° bis 100° C, vorzugsweise -10° bis 20° C, umgesetzt wird oder

d) die Verbindung der Formel Ia mit einem Aldehyd der Formel V zu einem Hydroxyderivat der Formel Ic bei Temperaturen von 0° bis 200° C, vorzugsweise 0° bis 160° C, umgesetzt wird:

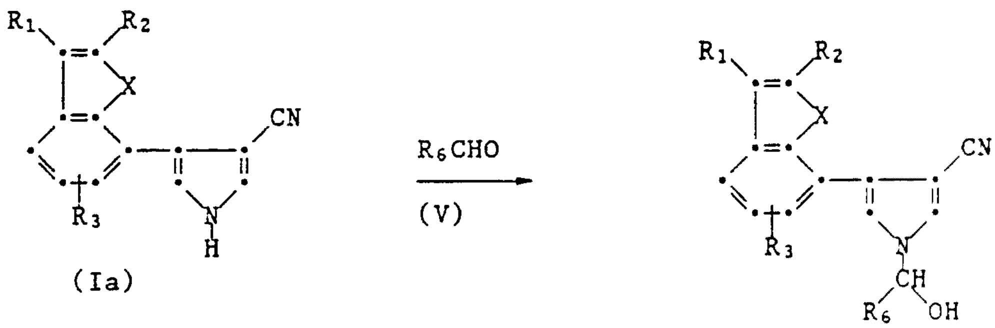


(Ia) (Ic)

und anschliessend an der Verbindung Ic die OH-Gruppe gegen einen anderen Rest Y ausgetauscht wird, indem eine Verbindung der Formel Ic entweder mit einer Säure der Formel VI

$R_7COOH$ (VI)

oder mit einem Säurehalogenid oder Säureanhydrid davon zu dem Acyloxyderivat der Formel Id

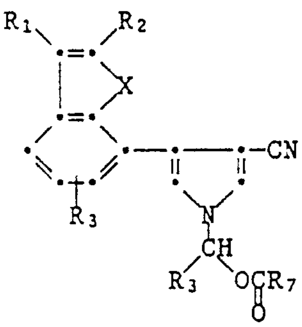

(Id)

in einem inerten Lösungsmittel umgesetzt wird,

oder zuerst die OH-Gruppe der Verbindung der Formel Ic gegen ein Halogenatom ausgetauscht wird, was eine Verbindung der Formel Ie

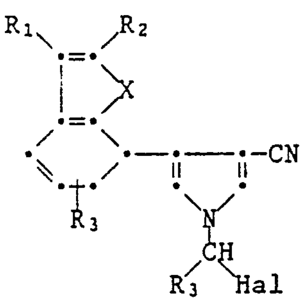

(Ie)

ergibt, und diese dann weiter durch Reaktion mit einem Salz der Formel VII

$R_7\text{-}COO^{\ominus} M'^{\oplus}$ (VII)

in einem inerten Lösungsmittel bei Temperaturen von 0° bis 150° C, vorzugsweise 20° bis 80° C, in eine Verbindung der Formel Id übergeführt wird, wobei Hal für Halogen, vorzugsweise Chlor und Brom, steht und $M'^{\oplus}$ ein Metallkation, vorzugsweise $Na^{\oplus}$, $K^{\oplus}$, $Mg^{\oplus\oplus}$ oder $Ca^{\oplus\oplus}$, darstellen, oder

e) die Verbindung der Formel Ia mit einer Verbindung der Formel VIII H-Z (VIII)

entweder in einem protischen Lösungsmittel bei Temperaturen von 0° bis 120° C, vorzugsweise 20° C bis zur Rückflusstemperatur des verwendeten Lösungsmittels, in Gegenwart eines basischen oder sauren Reaktionskatalysators mit Formaldehyd; oder in einem aprotischen Lösungsmittel in Gegenwart eines basischen Reaktionskatalysators bei Temperaturen von 0° bis 120° C, vorzugsweise 20° bis 80° C, mit 1,3,5-Trioxan oder Paraformaldehyd umgesetzt wird.

2. Verfahren gemäss Anspruch 1, dadurch gekennzeichnet, dass das N-Atom am Pyrrolrest der Verbindungen der Formel I einen der folgenden Reste trägt:

Wasserstoff oder CO-$R_4$, wobei $R_4$ unsubstituiertes $C_1$-$C_6$-Alkyl oder durch Halogen oder $C_1$-$C_3$-Alkoxy

substituiertes $C_1$-$C_6$-Alkyl; $C_3$-$C_6$-Alkenyl, $C_3$-$C_6$-Alkinyl, unsubstituiertes $C_1$-$C_6$-Alkoxy oder durch Halogen oder $C_1$-$C_3$-Alkoxy substituiertes $C_1$-$C_6$-Alkoxy; $C_3$-$C_6$-Alkenyloxy, $C_3$-$C_6$-Cycloalkyl oder Tetrahydrofur-2-yl darstellt.

3. Verfahren gemäss Anspruch 2, dadurch gekennzeichnet, dass das N-Atom am Pyrrolrest der Verbindungen der Formel I folgende Reste trägt:
Wasserstoff oder $COR_4$, wobei $R_4$ unsubstituiertes $C_1$-$C_4$-Alkyl oder durch Chlor, Brom oder $C_1$-$C_3$-Alkoxy substituiertes $C_1$-$C_4$-Alkyl; $C_3$-$C_4$-Alkenyl, $C_3$-$C_4$-Alkinyl, unsubstituiertes $C_1$-$C_4$-Alkoxy, oder durch Chlor, Brom oder $C_1$-$C_3$-Alkoxy substituiertes $C_1$-$C_4$-Alkoxy; $C_3$-$C_4$-Alkenyloxy, $C_3$-$C_6$-Cycloalkyl oder Tetrahydrofur-2-yl, darstellt.

4. Verfahren gemäss Anspruch 3, dadurch gekennzeichnet, dass das N-Atom am Pyrrolrest ein Wasserstoffatom trägt.

5. Verfahren gemäss Anspruch 4, dadurch gekennzeichnet, dass $R_1$ Wasserstoff, Halogen, $C_1$-$C_2$-Alkyl oder $C_1$-$C_2$-Haloalkyl, $R_2$ Wasserstoff oder Halogen und $R_3$ Wasserstoff bedeuten.

6. Verfahren gemäss Anspruch 5, dadurch gekennzeichnet, dass $R_1$ Wasserstoff oder Halogen und $R_2$ Wasserstoff bedeuten.

7. Verfahren zur Herstellung des 3-(Brombenzothiophen-7-yl)-4-cyanopyrrols gemäss Anspruch 6.

8. Verfahren zur Herstellung des 3-(Benzothiophen7-yl)-4-cyanopyrrols gemäss Anspruch 6.